# EUROPEAN PATENT APPLICATION

(11) **EP 2 055 697 A1**
(43) Date of publication of application: **06.05.2009**
(21) Application number: 08164539.2
(22) Date of filing: 17.09.2008
(51) Int. Cl.: C07D 239/34, C07D 239/38, A61K 31/505, A61P 35/00

(54) **Design and synthesis of novel tubulin polymerization inhibitors: benzoylphenylurea (BPU) sulfur analogs**

(30) Priority: 17.09.2007 US 960122 P
(71) Applicant: Champions Biotechnology, Inc., Arlington, VA 22201 (US)
(72) Inventor: Khan, Saeed, R., Owling Mills, MD 21117 (US); Hallur, Gurulingappa, Owlings Mills, MD 21117 (US); Hidalgo, Manuel, Baltimore, MD 21230 (US); Jimeno, Antonio, Baltimore, MD 21224 (US)
(74) Representative: Vossius & Partner

(57) **Abstract**

This invention provides a series of BPU analogues such as those of formula (XXX); their synthesis and evaluated biological activity. BPU sulfur analogues were found to possess up to 20 fold increased potency, when compared to reference compound 1, in various cancerous cell lines.

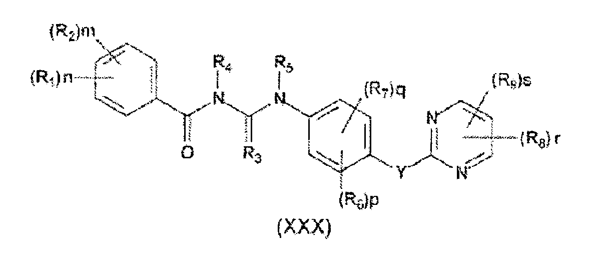

SEQUENCE LISTING

<110> Champions Biotechnology, Inc.

<120> DESIGN AND SYNTHESIS OF NOVEL TUBULIN POLYMERIZATION INHIBITORS:
      BENZOYLPHENYLUREA (BPU) SULFUR ANALOGS

<130> P2975 EP

<150> US 60/960,122
<151> 2007-09-17

<160> 22

<170> PatentIn version 3.3

<210> 1
<211> 19
<212> DNA
<213> Artificial sequence

<220>
<221> source
<223> /note="Description of artificial sequence:exon 9 forward primer"

<400> 1
ctgcctaacc cagtggtga                                                    19


<210> 2
<211> 19
<212> DNA
<213> Artificial sequence

<220>
<221> source
<223> /note="Description of artificial sequence: exon 9 reverse primer"

<400> 2
cacagtccca cgactccac                                                    19


<210> 3
<211> 20
<212> DNA
<213> Artificial sequence

<220>
<221> source
<223> /note="Description of artificial sequence: exon 10 forward
      primer"

<400> 3
ctctgccaag tccgaaagtg                                                   20


<210> 4
<211> 19
<212> DNA
<213> Artificial sequence

<220>
<221> source
<223> /note="Description of artificial sequence: exon 10 reverse

primer"

<400> 4
tcctgagagc ccaagaagg 19


<210> 5
<211> 20
<212> DNA
<213> Artificial sequence

<220>
<221> source
<223> /note="Description of artificial sequence: exon 11 forward
      primer"

<400> 5
ataagggcct gggcttacac 20


<210> 6
<211> 20
<212> DNA
<213> Artificial sequence

<220>
<221> source
<223> /note="Description of artificial sequence: exon 11 reverse
      primer"

<400> 6
ccgcaagttt cacactcaac 20


<210> 7
<211> 20
<212> DNA
<213> Artificial sequence

<220>
<221> source
<223> /note="Description of artificial sequence: exon 12 forward
      primer"

<400> 7
ttcaagtcct gctgcaaacc 20


<210> 8
<211> 20
<212> DNA
<213> Artificial sequence

<220>
<221> source
<223> /note="Description of artificial sequence: exon 12 reverse
      primer"

<400> 8
ctccaggtga ttgagacagg 20


<210> 9
<211> 20

<212>  DNA
<213>  Artificial sequence

<220>
<221>  source
<223>  /note="Description of artificial sequence: exon 13 forward
       primer"

<400>  9
aggctgcagt gaggtgagat                                                    20


<210>  10
<211>  20
<212>  DNA
<213>  Artificial sequence

<220>
<221>  source
<223>  /note="Description of artificial sequence: exon 13 reverse
       primer"

<400>  10
ccgagtgaca aaagcaggtt                                                    20


<210>  11
<211>  20
<212>  DNA
<213>  Artificial sequence

<220>
<221>  source
<223>  /note="Description of artificial sequence: exon 1 forward primer"

<400>  11
tacatgaccg gcatcgacta                                                    20


<210>  12
<211>  20
<212>  DNA
<213>  Artificial sequence

<220>
<221>  source
<223>  /note="Description of artificial sequence: exon 1 reverse primer"

<400>  12
acaaacttga gaggggcaaa                                                    20


<210>  13
<211>  20
<212>  DNA
<213>  Artificial sequence

<220>
<221>  source
<223>  /note="Description of artificial sequence: exons 2 and 3 forward
       primer"

<400>  13
agcgagactc cgtctcaaaa                                                    20

<210> 14
<211> 20
<212> DNA
<213> Artificial sequence

<220>
<221> source
<223> /note="Description of artificial sequence: exons 2 and 3 reverse
      primer"

<400> 14
cttttgctgt gtccttgcac                                                    20


<210> 15
<211> 23
<212> DNA
<213> Artificial sequence

<220>
<221> source
<223> /note="Description of artificial sequence: exon 4a forward
      primer"

<400> 15
cgagggaatt atttgaaaag ttg                                                23


<210> 16
<211> 18
<212> DNA
<213> Artificial sequence

<220>
<221> source
<223> /note="Description of artificial sequence: exon 4a reverse
      primer"

<400> 16
ggcatcgaag acctgctg                                                      18


<210> 17
<211> 20
<212> DNA
<213> Artificial sequence

<220>
<221> source
<223> /note="Description of artificial sequence: exon 4b forward
      primer"

<400> 17
ttctttatgc ctggctttgc                                                    20


<210> 18
<211> 20
<212> DNA
<213> Artificial sequence

<220>

<221>  source
<223>  /note="Description of artificial sequence: exon 4b reverse
      primer"

<400>  18
cccagaatgg cagaaaccta                                                    20


<210>  19
<211>  21
<212>  DNA
<213>  Artificial sequence

<220>
<221>  source
<223>  /note="Description of artificial sequence: exon 4c forward
      primer"

<400>  19
tccctcagaa tttgtgtttg c                                                  21


<210>  20
<211>  20
<212>  DNA
<213>  Artificial sequence

<220>
<221>  source
<223>  /note="Description of artificial sequence: exon 4c reverse
      primer"

<400>  20
gctcgagtga gggaggtaga                                                    20


<210>  21
<211>  24
<212>  DNA
<213>  Artificial sequence

<220>
<221>  source
<223>  /note="Description of artificial sequence: exon 4d forward
      primer"

<400>  21
tgtcagcagt attatctcca cttt                                               24


<210>  22
<211>  21
<212>  DNA
<213>  Artificial sequence

<220>
<221>  source
<223>  /note="Description of artificial sequence: exon 4d reverse
      primer"

<400>  22
ttaagtgtca atttaaggag a                                                  21

## Description

### GOVERNMENT INTEREST STATEMENT

This invention was made in part with government support under grant number #NO1-CO-12400 awarded by the National Cancer Institute and all joint inventors have assigned their rights to Johns Hopkins University. The government may have certain rights in the invention.

### FIELD OF INVENTION

Benzoylphenylurea (BPU) sulfur analogues potency in treating various cancer types.

### BACKGROUND OF THE INVENTION

The present invention relates to design and synthesis of novel benzoylphenylurea (BPU) and benzoylphenylthiourea (BPTU) oxygen, sulfur, sulfoxide and sulfonyl analogs with increased potency and enhanced water solubility. More specifically, the present invention is directed toward the synthesis of sulfur, hydroximic acid, and boronic acid analogs of BPU.

The lack of selectivity of many cancer agents and the occurrence of intrinsic or acquired resistance of tumors to chemotherapy has been major obstacles in the treatment of cancer. Microtubules, which are key components of the cell, play an important role in a variety of cellular process, including mitosis and cell division. Antimitotic agents are divided into two major classes: (1) microtubule stabilizers such as paclitaxel and docetaxel, which prevent the depolymerization of tubulin.

Although some of these agents have been used as antiproliferative agents in the treatment of human malignancies, they suffer from drug resistance mediated through the expression of efflux pumps.

Benzoylphenylurea (BPU) compounds were originally developed as insecticides, and their antitumor activity was found during random screening. Various analogues of BPU were synthesized, and their cytotoxic activity was examined to establish structure-activity relationships (Okada, H., et al., Chem. Pharm. Bull., 39:2308-2315 (1991)). To improve physicochemical properties, organic and water soluble BPU derivatives were developed. See, Okada, H., et al., Chem. Pharm. Bull., 42:57-61 (1994); and Okada, H., et al., Chem. Pharm. Bull, 47:430-433 (1999). Six of these analogues were screened at the NCI for their cytotoxicity against various cancer cell lines. They exhibited potent antitumor activity in vitro against several cancer cell lines, as well in vivo against several tumor models, These compounds also have been reported to be effective inhibitors of tubulin polymerization (Holligshead, M. G., et al., Proc. Am. Assoc, Cancer Res., 39:164 (1979)). One of these agents, compound 1 (NSC-639829), (Holligshead, M. G., et al., Proc. Am.Assoc. Cancer Res., 39:164 (1979)) is currently being evaluated in Phase I clinical trials in patients with refractory metastatic cancer. See, Messersmith, W. A., et al., Proc. Am. Soc. Clin. Oncol., 22:203 (2003); and Edelman, M. J., et al., Proc. Am. Soc. Clin. Oncol., 22:137 (2003). It was previously reported the synthesis and antitumor evaluation of a set of BPU analogues (Gurulingappa, H., et al., Bioorg. Med. Chem. Lett., 14:2213-2216 (2004)).

There is still a need to improve physicochemical properties, organic and water soluble BPU derivatives. To that end the present invention discloses a novel series of synthesized derivatives of BPU by replacing the urea moiety with thiourea and the ether linkage with sulfide, sulfoxide, or sulfone groups. The activity of such novel agents, are significantly more toxic to cancer cells in culture.

### SUMMARY OF THE INVENTION

This invention provides, in one embodiment, a compound represented by the structure of formula XXX: 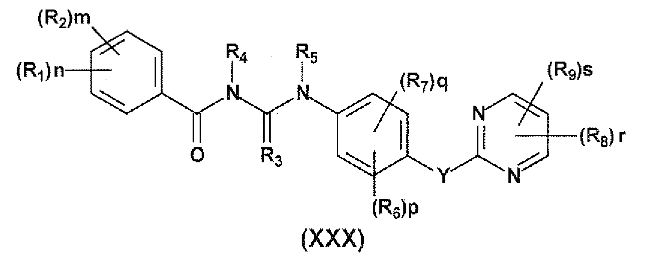 wherein
R₁, and R₂ are independently H, NH₂, NO₂, Cl, Br, I, CN, NHMe, NMe₂, Me, CF₃, COOH, CONHOH, alkyl or aryl;
R₆ and R₇ are independently H, NH₂, NO₂, F, Cl, Br, I, CN, NHMe, NMe₂, CF₃, COOH, CONHOH, alkyl or aryl;
Y is S, SO or SO₂;
R₃ is O or S;
R₄ and R₅ are independently, H ,alkyl, aryl or -SR₁₀, wherein R₁₀ is a substituted alkyl, cycloalkyl, aryl, heterocycle, or substituted phenyl fused with a saturated or unsaturated 4- to 6- membered rings optionally containing one to three heteroatoms independently selected from N, O, and S;
R₈ and R₉ are independently H, alkyl or halogen;
n is an integer between 1-5;
m is an integer between 0-4;
r is an integer between 1-3;
s is an integer between 0-2;
q is an integer between 1-4; and
p is an integer between 0-3.

In another embodiment, the present invention provides a compound represented by the structure of formula XXXI: 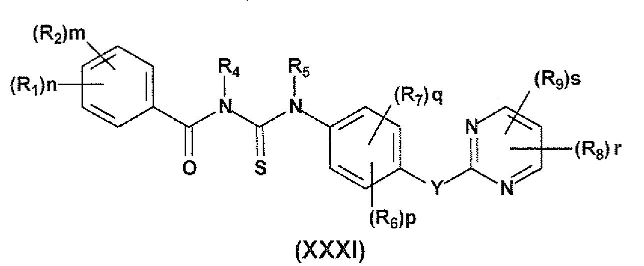 wherein R₁ and R₂, are independently H, NH₂, NO₂, F, Cl, Br, I, CN, NHMe, NMe₂, Me, CF₃, COOH, CONHOH, alkyl or aryl;
R₆ and R₇ are independently H, NH₂, NO₂, F, Br, I, CN, NHMe, NMe₂, CF₃, COOH, CONHOH, alkyl or aryl;
Y is S, SO, SO₂ or O;
R₄ and R₅ are independently, H ,alkyl, aryl or -SR₁₀, wherein R₁₀ is a substituted alkyl, cycloalkyl, aryl, heterocycle, or substituted phenyl fused with a saturated or unsaturated 4- to 6- membered rings optionally containing one to three heteroatoms independently selected from N, O, and S;
R₈ and R₉ are independently H, alkyl or halogen;
n is an integer between 1-5;
m is an integer between 0-4;
r is an integer between 1-3;
s is an integer between 0-2;
q is an integer between 1-4; and
p is an integer between 0-3.

A compound represented by the structure of formula XXXII: 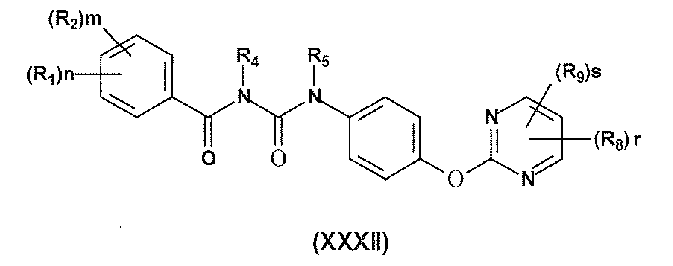 wherein,
R₁ is NH₂, NHMe, NMe₂, or NO₂ and R₂ is independently H, NH₂, NO₂, CN, NHMe, NMe₂, CF₃, COOH, CONHOH, alkyl or aryl;
R₄ and R₅ are independently, H, alkyl, aryl or -SR₁₀, wherein R₁₀ is a substituted alkyl, cycloalkyl, aryl, heterocycle, or substituted phenyl fused with a saturated or unsaturated 4- to 6- membered rings optionally containing one to three heteroatoms independently selected from N, O, and S;
R₈ and R₉ are independently H, alkyl or halogen;
n is an integer between 1-5;
m is an integer between 0-4;
r is an integer between 1-3;
s is an integer between 0-2; and
wherein if R₁ is NO₂ in the ortho position, n is 1, m is 0, s is 0, r is 1, R₄ and R₅ are H, than R₈ is not iodine, Br or H in the para position

### BRIEF DESCRIPTION OF THE DRAWINGS

The accompanying drawings, which are incorporated in and form a part of the specification, illustrate the preferred embodiments of the present invention, and together with the description serve to explain the principles of the invention.

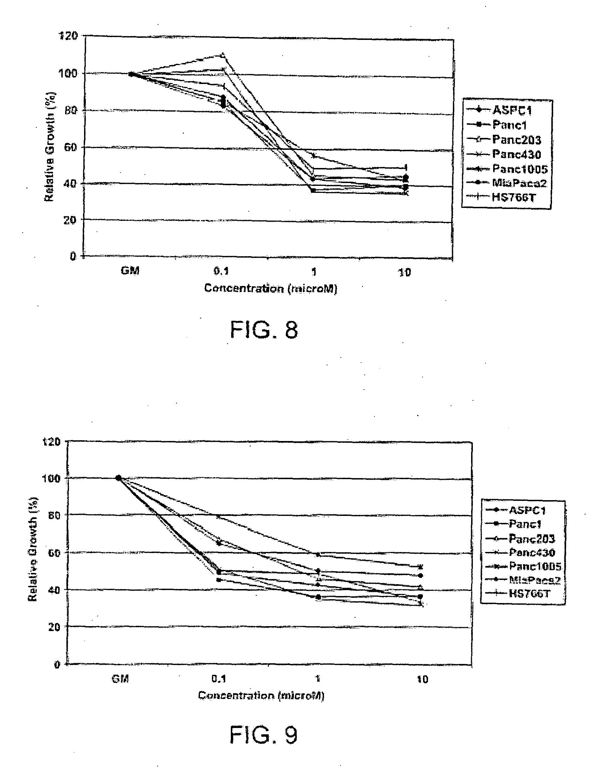

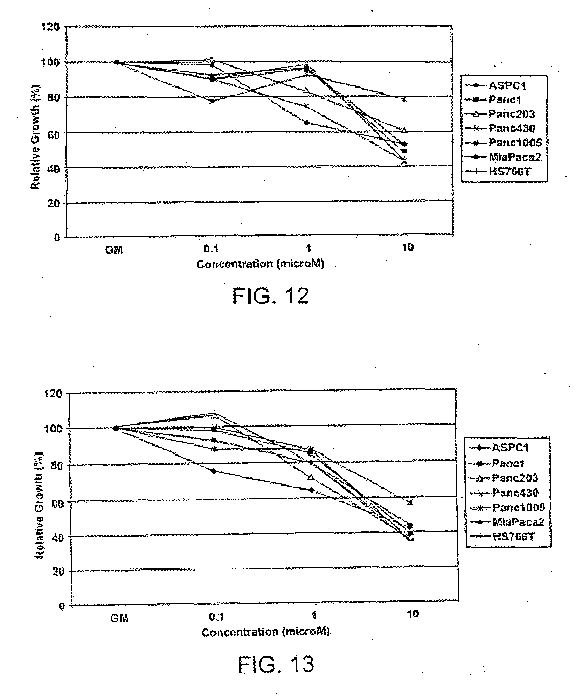

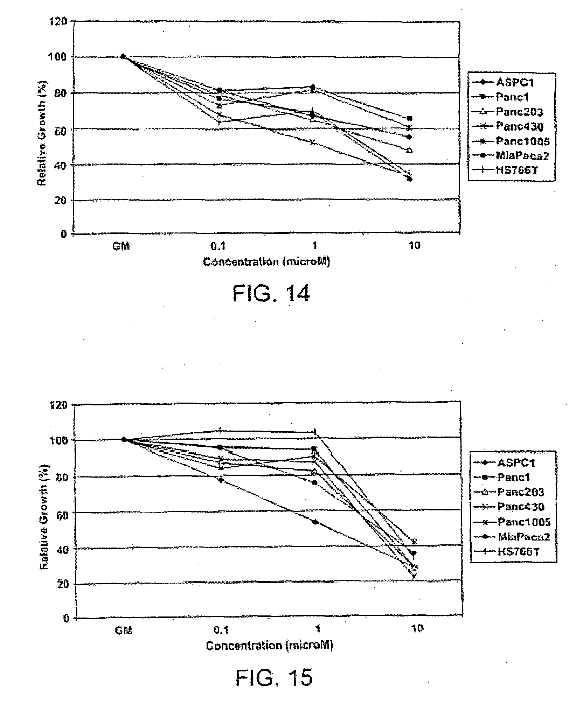

In the drawings, Figures 8-16, the cytotoxic effects of the test compounds on seven pancreatic cell lines was measured. The horizontal axis depicts various dilutions of the test compounds that were exposed to pancreatic cell lines. The vertical axis (cell number) depicts the number of pancreatic cells present after exposure to a specific concentration of the tested compound as compared to the cell number at time zero.

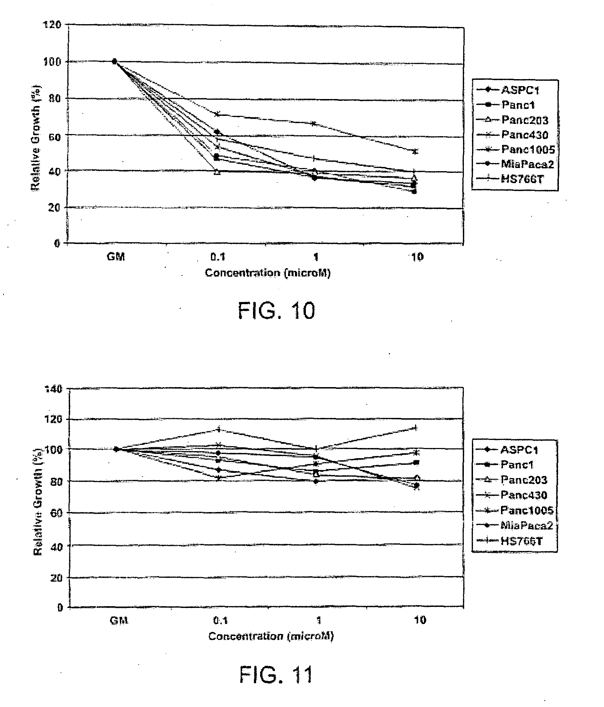

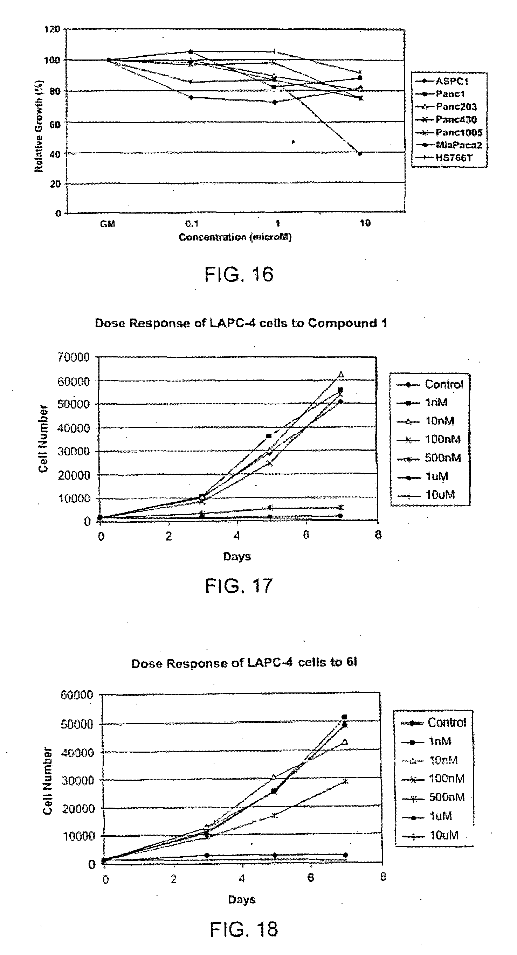

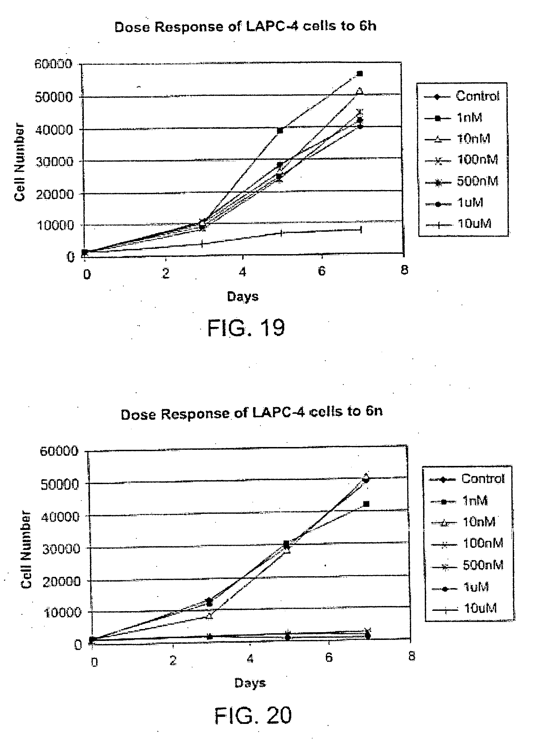

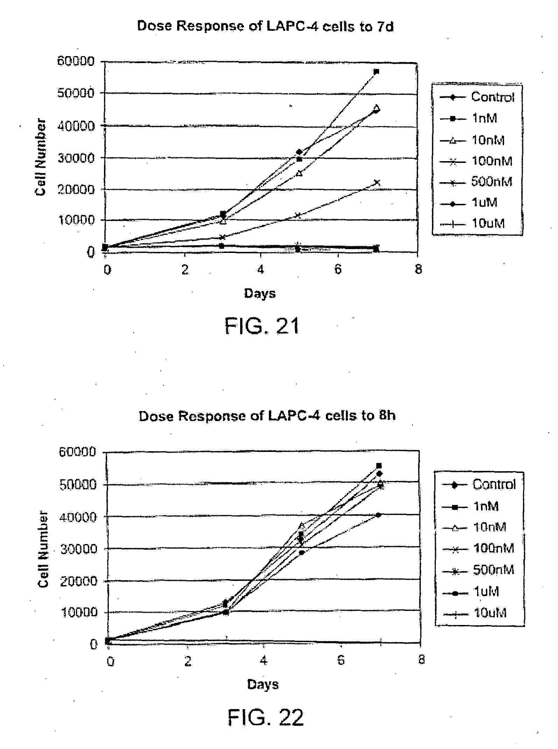

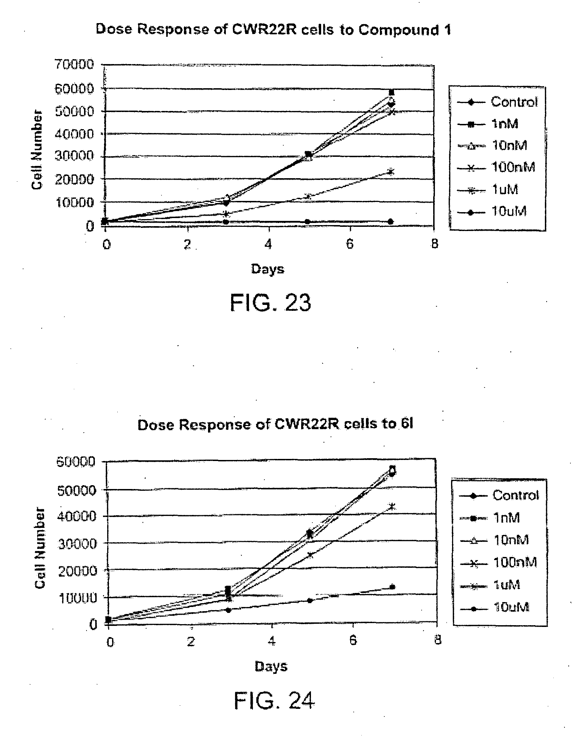

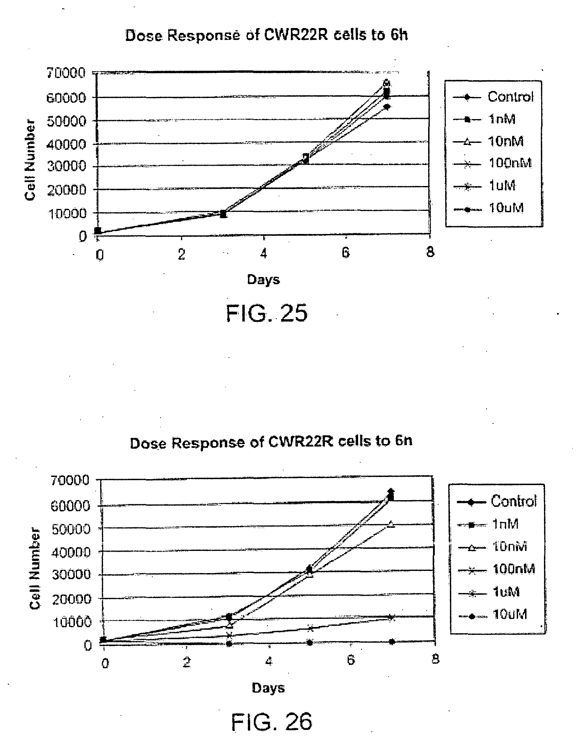

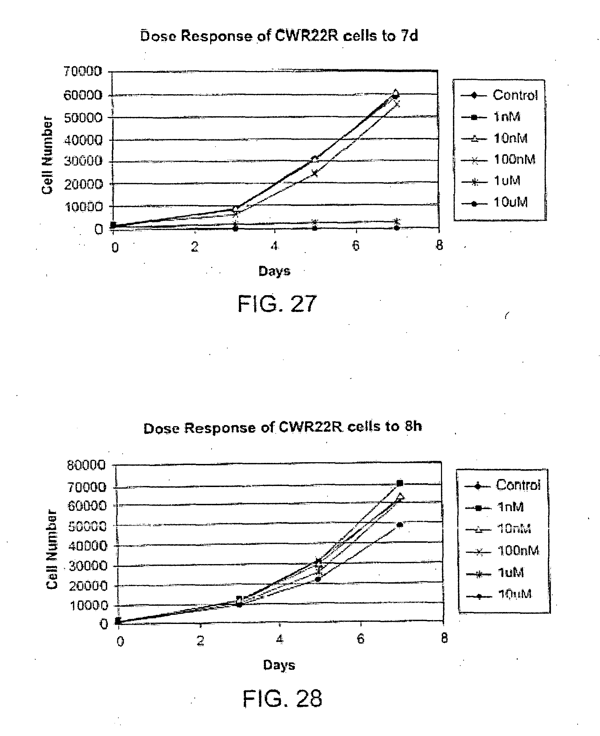

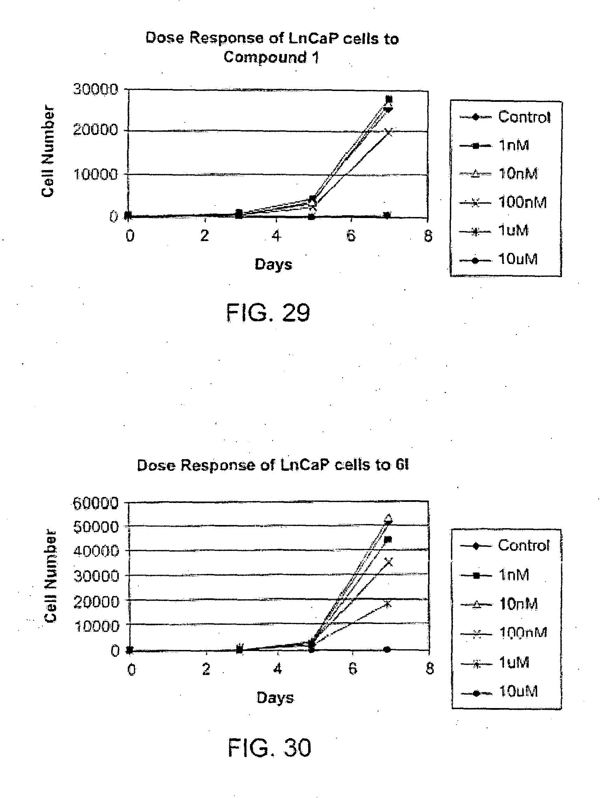

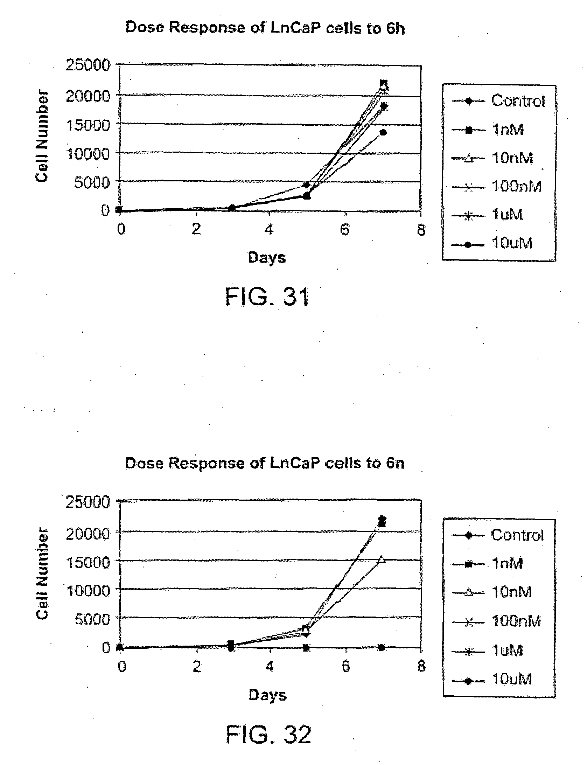

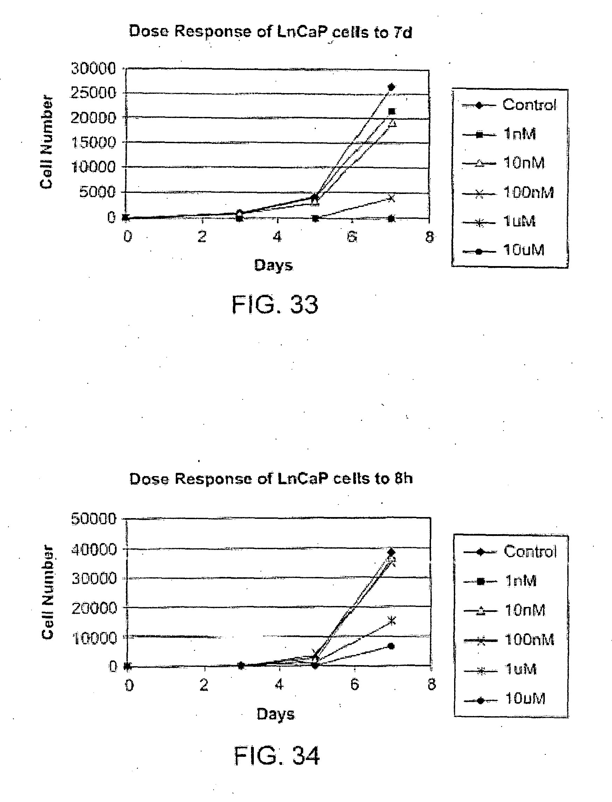

In the drawings, Figures 17-34, the growth inhibition effects of the test compounds on prostate cancer cell lines LAPC-4 (figures 10-15); CWR22R (figures 16-21); and LnCaP (figures 22-27) were measured. The horizontal axis depicts the days after exposure on which the assays were performed. The vertical axis (cell number) depicts the number of pancreatic cells present after exposure to a specific concentration of the tested compound as compared to the cell number at time zero.

**Figure 1** schematically depicts the synthesis scheme for compounds of the present invention.

**Figure 2** schematically depicts the synthesis of the Ring-A aniline modified amino acids prodrugs.

**Figure 3** schematically depicts the synthesis scheme for Ring-A aniline modified carbamylosulfenyl derivative of BPU sulfur analogs.

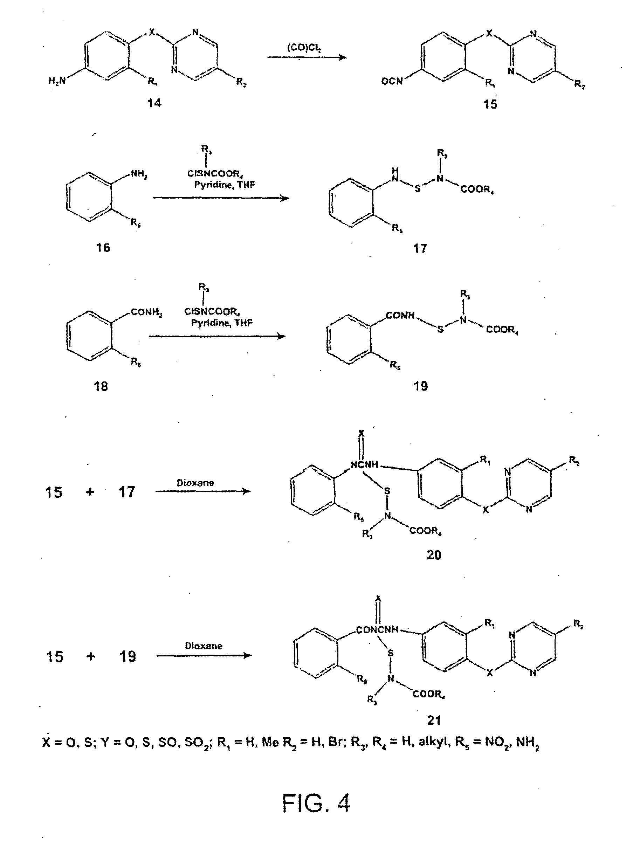

**Figure 4** schematically depicts the synthesis scheme for compounds 4a and 4b of the present invention.

**Figure 5** schematically depicts the synthesis scheme for compounds of the present invention.

**Figure 6** schematically depicts the synthesis scheme for both Ring-A and urea modified prodrugs of the present invention.

**Figure 7** is a bar graph demonstrating the apoptosis of MCF-7 cells by compounds 6n and 1.

**Figure 8** depicts the dose response curves generated by exposing seven pancreatic cell lines to various concentrations of compound 1 as a control.

**Figure 9** depicts the dose response curves generated by exposing seven pancreatic cell lines to various concentrations of compound 6n of the present invention.

**Figure 10** depicts the dose response curves generated by exposing seven pancreatic cell lines to various concentrations of compound 7d of the present invention.

**Figure 11** depicts the dose response curves generated by exposing seven pancreatic cell lines to various concentrations of compound 6d of the present invention.

**Figure 12** depicts the dose response curves generated by exposing seven pancreatic cell lines to various concentrations of compound 6h of the present invention.

**Figure 13** depicts the dose response curves generated by exposing seven pancreatic cell lines to various concentrations of compound 7b of the present invention.

**Figure 14** depicts the dose response curves generated by exposing seven pancreatic cell lines to various concentrations of compound 6g of the present invention.

**Figure 15** depicts the dose response curves generated by exposing seven pancreatic cell lines to various concentrations of compound 8g of the present invention.

**Figure 16** depicts the dose response curves generated by exposing seven pancreatic cell lines to various concentrations of compound 8h of the present invention.

**Figure 17** depicts the dose response curves generated by exposing various concentrations of compound 1 of the present invention to human prostate cancer cell line LAPC-4 versus a control using only a solvent.

**Figure 18** depicts the dose response curves generated by exposing various concentrations of compound 61 of the present invention to human prostate cancer cell line LAPC-4 versus a control using only a solvent.

**Figure 19** depicts the dose response curves generated by exposing various concentrations of compound 6h of the present invention to human prostate cancer cell line LAPC-4 versus a control using only a solvent.

**Figure 20** depicts the dose response curves generated by exposing various concentrations of compound 6n of the present invention to human prostate cancer cell line LAPC-4 versus a control using only a solvent.

**Figure 21** depicts the dose response curves generated by exposing various concentrations of compound 7d of the present invention to human prostate cancer cell line LAPC-4 versus a control using only a solvent.

**Figure 22** depicts the dose response curves generated by exposing various concentrations of compound 8h of the present invention to human prostate cancer cell line LAPC-4 versus a control using only a solvent.

**Figure 23** depicts the dose response curves generated by exposing various concentrations of compound 1 of the present invention to human prostate cancer cell line CWR22R versus a control using only a solvent.

**Figure 24** depicts the dose response curves generated by exposing various concentrations of compound 61 of the present invention to human prostate cancer cell line CWR22R versus a control using only a solvent.

**Figure 25** depicts the dose response curves generated by exposing various concentrations of compound 6h of the present invention to human prostate cancer cell line CWR22R versus a control using only a solvent.

**Figure 26** depicts the dose response curves generated by exposing various concentrations of compound 6n of the present invention to human prostate cancer cell line CWR22R versus a control using only a solvent.

**Figure 27** depicts the dose response curves generated by exposing various concentrations of compound 7d of the present invention to human prostate cancer cell line CWR22R versus a control using only a solvent.

**Figure 28** depicts the dose response curves generated by exposing various concentrations of compound 8h of the present invention to human prostate cancer cell line CWR22R versus a control using only a solvent.

**Figure 29** depicts the dose response curves generated by exposing various concentrations of compound 1 of the present invention to human prostate cancer cell line LnCaP versus a control using only a solvent.

**Figure 30** depicts the dose response curves generated by exposing various concentrations of compound 61 of the present invention to human prostate cancer cell line LnCaP versus a control using only a solvent.

**Figure 31** depicts the dose response curves generated by exposing various concentrations of compound 6h of the present invention to human prostate cancer cell line LnCaP versus a control using only a solvent.

**Figure 32** depicts the dose response curves generated by exposing various concentrations of compound 6n of the present invention to human prostate cancer cell line LnCaP versus a control using only a solvent.

**Figure 33** depicts the dose response curves generated by exposing various concentrations of compound 7d of the present invention to human prostate cancer cell line LnCaP versus a control using only a solvent.

**Figure 34** depicts the dose response curves generated by exposing various concentrations of compound 8h of the present invention to human prostate cancer cell line LnCaP versus a control using only a solvent.

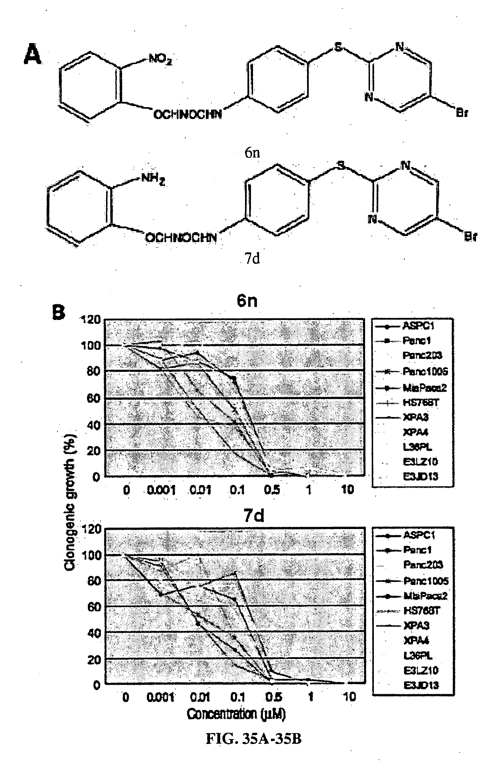

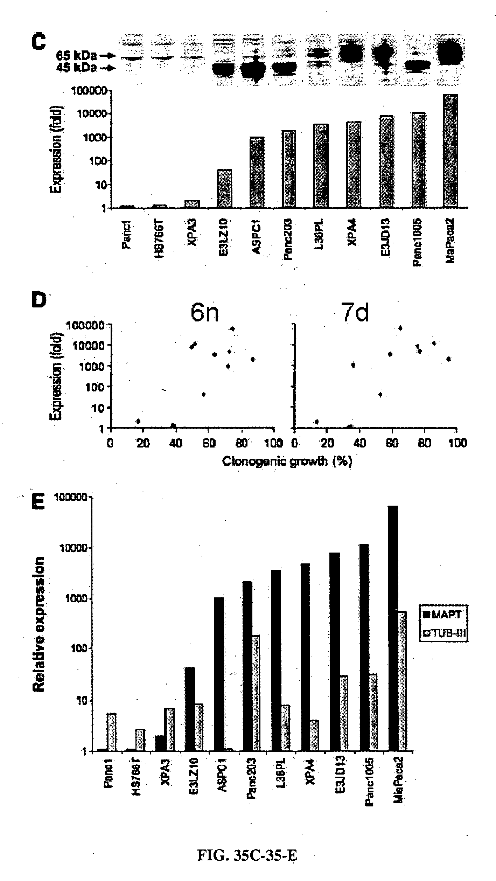

**Figure 35A** depicts a chemical structure of compounds 6n and 7d. **Figure 35B** depicts a graph showing the clonogenic growth after exposure to compounds 6n and 7d for 96 h. Two cell lines had their clonogenic capability significantly impaired by 6n in the 1 to 10 nmol/L range (HS766T and XPA3) and three cell lines (Panc1, XPA3, and ASPC1) by 7d. With both drugs, the most resistant cell line was E3JD 13, with which there was a low level of colony viability at concentrations of ≥ 1 µmol/L. **Figure 35C** depicts a bar graph and a gel showing MAPT mRNA levels varied across a wide range (65,000-fold). There was a significant correlation between mRNA and protein, with the three lowest mRNA-expressing cells lines also showing low protein expression. Two isoforms of MAPT protein were detected (45 and 65 kDa), with a distinct distribution. The cell lines with lowest mRNA expression showed traces of the 65-kDa isoform, whereas there was a degree of polarity in the other except L36PL that expressed both. In the high protein-expressing cell lines, no correlation between isoformexpression and activity was detected. **Figure 35D** depicts graphs showing the correlation between clonogenic growth (X-axis) at 0.1 µmol/L and MAPT mRNA levels (Y-axis) in the panel of cell lines. **Figure 35E** depicts a bar graph showing the comparison of the expression of class III β-tubulin and MAPT in the panel of cell lines.

**Figure 36** depicts in-vivo growth measurements of pancreas cancer xenografts treated with the study drugs (compounds 7d and 6n). **Figure 36A** depicts a bar graph showing compounds 7d and 6n rates of growth inhibition and MAPT expression. **Figure 36B** depicts a bar graph comparing the efficacy of compounds 7d, 6n and docetaxel. P <0.05, compared with control (Student's t test).

**Figure 37** shows integrated analysis of all the data points from nine treatment groups for compound 6n (**Figure 37A****)** and 7d **Figure 37B****.** Black dots, control tumors; gray dots, treated tumors.

### DETAILED DESCRIPTION OF THE INVENTION

In one embodiment, the abbreviations used herein have their conventional meaning within the chemical and biological arts. In another embodiment, moieties are specified by their conventional chemical formulae, written from left to right, they equally encompass the chemically identical moieties that would result from writing the structure from right to left, e.g., -CH₂O- is equivalent to -OCH₂-.

In another embodiment, the term "alkyl," by itself or as part of another substituent, means, unless otherwise stated, a straight (i.e. unbranched) or branched chain, or cyclic hydrocarbon radical, or combination thereof, which may be fully saturated, mono- or polyunsaturated and can include di- and multivalent radicals, having the number of carbon atoms designated (i.e. C1-C10 means one to ten carbons). In another embodiment, examples of saturated hydrocarbon radicals include, but are not limited to, groups such as methyl, ethyl, n- propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. In another embodiment, an unsaturated alkyl group is one having one or more double bonds or triple bonds. In another embodiment, unsaturated alkyl groups include, but are not limited to, vinyl, 2-propenyl, crotyl, 2- isopentenyl, 2-(butadienyl), 2,4- pentadienyl, 3-(1,4-pentadienyl), ethynyl, 1- and 3- propynyl, 3-butynyl, and the higher homologs and isomers.

In another embodiment, the term "alkylene" by itself or as part of another substituent means adivalent radical derived from an alkyl, as exemplified, but not limited, by -CH₂CH₂CH₂CH₂-. In another embodiment, an alkyl (or alkylene) group will have from 1 to 24 carbon atoms, including those groups having 10 or fewer carbon atoms. In another embodiment, a "lower alkyl" or "lower alkylene" is a shorter chain alkyl or alkylene group, generally having eight or fewer carbon atoms.

In another embodiment, the terms "alkoxy," "alkylamino," and "alkylthio" (or thioalkoxy) are used in their conventional sense, and refer to those alkyl groups attached to the remainder of the molecule via an oxygen atom, an amino group, or a sulfur atom, respectively.

In another embodiment, the term "heteroalkyl," by itself or in combination with another term, means, unless otherwise stated, a stable straight or branched chain, or cyclic hydrocarbon radical, or combinations thereof consisting of the stated number of carbon atoms and a hetero atom selected from the group consisting of O, N, P, Si and S, and wherein the nitrogen and sulfur atoms may optionally be oxidized and the nitrogen heteroatom may optionally be quaternized. In another embodiment, the heteroatom(s) O, N, P and S and Si may be placed at any interior position of the heteroalkyl group or at the position at which the alkyl group is attached to the remainder of the molecule. Examples include, but are not limited to, -CH₂-CH₂-O-CH₃, -CH₂- CH₂-NH-CH₃, -CH₂-CH₂-N(CH₃)-CH₃, -CH₂-S-CH₂-CH₃, -CH₂-CH₂,-S(O)-CH₃, -CH₂-CH₂-S(O)₂-CH₃, -CH=CH-O-CH₃, -Si(CH3)₃, -CH₂-CH=N-OCH₃, - CH=CH-N(CH₃)-CH₃, O-CH₃, -O-CH₂-CH₃, and -CN. In another embodiment, up to two heteroatoms may be consecutive, such as, for example, -CH₂-NH-OCH₃ and -CH₂-O-Si(CH₃)₃. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-.

In another embodiment, for heteroalkylene groups, heteroatoms can also occupy either or both of the chain termini (e.g., alkyleneoxy, alkylenedioxy, alkyleneamino, alkylenediamino, and the like). In another embodiment, for alkylene and heteroalkylene linking groups, no orientation of the linking group is implied by the direction in which the formula of the linking group is written. In another embodiment, the formula -C(O)₂R'- represents both -C(O)₂R'- and -R'C(O) 2-. In another embodiment, heteroalkyl groups, as used herein, include those groups that are attached to the remainder of the molecule through a heteroatom, such as -C(O)R', -C(O)NR', -NR'R", -OR', - SR', and/or -SO₂R'. In another embodiment, "heteroalkyl" is recited, followed by recitations of specific heteroalkyl groups, such as -NR'R" or the like, it will be understood that the terms heteroalkyl and -NR'R" are not redundant or mutually exclusive. In another embodiment, specific heteroalkyl groups are recited to add clarity. In another embodiment, the term "heteroalkyl" should not be interpreted herein as excluding specific heteroalkyl groups, such as - NR'R" or the like.

In another embodiment, the terms "cycloalkyl" and "heterocycloalkyl", by themselves or in combination with other terms, represent, unless otherwise stated, cyclic versions of "alkyl" and "heteroalkyl", respectively. In another embodiment, for heterocycloalkyl, a heteroatom can occupy the position at which the hetero cycle is attached to the remainder of the molecule.

In another embodiment, cycloalkyl include, but are not limited to, cyclopentyl, cyclohexyl, 1-cyclohexenyl, 3-cyclohexenyl, cycloheptyl, and the like. In another embodiment, heterocycloalkyl include, but are not limited to, 1-(1, 2, 5, 6-tetrahydropyridyl), 1-piperidinyl, 2-piperidinyl, 3-piperidinyl, 4-morpholinyl, 3-morpholinyl, tetrahydrofiiran-2-yl, tetrahydrofuran-3-yl, tetrahydrothien-2-yl, tetrahydrothien-3-yl, 1-piperazinyl, 2-piperazinyl, and the like.

In another embodiment, the terms "halo" or "halogen," by themselves or as part of another substituent, mean, unless otherwise stated, a fluorine, chlorine, bromine, or iodine atom. In another embodiment, terms such as "haloalkyl," are meant to include monohaloalkyl and polyhaloalkyl. In another embodiment, the term "halo(C1-C4)alkyl" is mean to include, but not be limited to, trifluoromethyl, 2,2,2-trifluoroethyl, 4-chiorobutyl, 3-bromopropyl, and the like.

In another embodiment, the term "aryl" means, unless otherwise stated, a polyunsaturated, aromatic, hydrocarbon substituent which is a single ring or multiple rings (preferably from 1 to 3 rings) which are fused together or linked covalently. In another embodiment, the term "heteroaryl" refers to aryl groups (or rings) that contain from one to four heteroatoms selected from N, O, and S, wherein the nitrogen and sulfur atoms are optionally oxidized, and the nitrogen atom(s) are optionally quaternized. In another embodiment, a heteroaryl group is attached to the remainder of the molecule through a carbon or heteroatom. Non- limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. In another embodiment, substituent moieties for each of the above noted aryl and heteroaryl ring systems may be selected from the group of acceptable substituent moieties described below.

In another embodiment, the term "aryl" when used in combination with other terms (e. g., aryloxy, aryithioxy, arylalkyl) includes both aryl and heteroaryl rings as defined above. In another embodiment, the term "arylalkyl" is meant to include those radicals in which an aryl group is attached to an alkyl group (e.g., benzyl, phenethyl, pyridylmethyl and the like) including those alkyl groups in which a carbon atom (e.g., a methylene group) has been replaced by, for example, an oxygen atom (e.g, phenoxymethyl, 2-pyridyloxymethyl, 3-(1-naphthyloxy)propyl, and the like).

In another embodiment, the term "oxo" as used herein means an oxygen that is double bonded to a carbon atom. In another embodiment, each ofthe above terms (e.g., "alkyl," "heteroalkyl," "aryl" and "heteroaryl") are meant to include both substituted and unsubstituted forms of the indicated radical. Preferred substituent moieties for each type of radical are provided below.

In another embodiment, substituent moieties for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) is one or more of a variety of groups selected from, but not limited to: -OR', =O, =NR', =N-OR', -NR'R", -SR', -halogen, -SiR'R"R'", -OC(O)R', - C(O)R', -CO₂R', -CONR'R", -OC(O) NR'R", -NR"C(O)R', - NR'-C(O)NR"R"', -NR"C(O)₂R', -NR-C(NR'R"R")=NR"", -NR-C(NR'R")=NR"', -S(O)R', -S(O)₂R', -S(O)₂NR'R", -NRSO₂R', -CN and -NO₂ in a number ranging from zero to (2m'+1), where m' is the total number of carbon atoms in such radical. In another embodiment, R', R", R" and R"" each preferably independently refer to hydrogen, substituted or unsubstituted heteroalkyl, substituted or unsubstituted cycloalkyl, substituted or unsubstituted heterocycloalkyl, substituted or unsubstituted aryl (e.g., aryl substituted with 1-3 halogens), substituted or unsubstituted alkyl, alkoxy or thioalkoxy groups, or arylalkyl groups. In another embodiment, a compound of the invention includes more than one R group, for example, each of the R groups is independently selected as are each R', R", R" and R"" groups when more than one of these groups is present. In another embodiment, when R' and R" are attached to the same nitrogen atom, they are combined with the nitrogen atom to form a 4-, 5-, 6-, or 7-membered ring. In another embodiment, NR'R" is meant to include, but not be limited to, 1 -pyrrolidinyl and 4- morpholinyl. In another embodiment, one of skill in the art will understand that the term "alkyl" is meant to include groups including carbon atoms bound to groups other than hydrogen groups, such as haloalkyl (e.g., -CF₃ and - CH₂CF₃) and acyl (e.g., -C(O)CH₃, -C(O)CF₃, -C(O)CH₂OCH₃, and the like).

In another embodiment, similar to the substituent moieties described for the alkyl radical, substituent moieties for the aryl and heteroaryl groups are varied and may be selected from, for example: halogen, OR', -NR'R", -SR', -halogen, -SiR'R''R''', OC(O)R', -C(O)R', CO2R', -CONR'R", OC(O)NR'R", -NR"C(O)R', NR' C(O)NR"R"', -NR"C(O)₂R', NR-C(NR'R"R'")=NR"", NR C(NR'R")=NR'", -S(O)R', -S(O)2R', -S(O)₂NR'R", NRSO₂R', -CN and -NO₂, -R', -N₃, CH(Ph)₂, fluoro(C1-C4)alkoxy, and fluoro(C1-C4)alkyl, in a number ranging from zero to the total number of open valences on the aromatic ring system; and where R', R", R"' and R"" are preferably independently selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. In another embodiment, a compound of the invention includes more than one R group, for example, each of the R groups is independently selected as are each R', R", R''' and R"" groups when more than one of these groups is present.

In another embodiment, two of the substituent moieties on adjacent atoms of the aryl or heteroaryl ring may optionally form a ring of the formula T-C(O)-(CRR')q-U-, wherein T and U are independently -NR-, -O-, -CRR'- or a single bond, and q is an integer of from 0 to 3. In another embodiment, two of the substituent moieties on adjacent atoms of the aryl or heteroaryl ring may optionally be replaced with a substituent of the formula A-(CH2)r-B-, wherein A and B are independently -CRR'-, -O-, -NR-, -S-, -S(O)-, S(O)2-, -S(O)2NR'- or a single bond, and r is an integer of from 1 to 4. In another embodiment, one of the single bonds of the new ring so formed may optionally be replaced with a double bond. In another embodiment, two of the substituent moieties on adjacent atoms of the aryl or heteroaryl ring may optionally be replaced with a substituent ofthe formula -(CRR')s-X'-(C"R'")d-, where s and d are independently integers of from 0 to 3, and X' is-O-, -NR'-, -S-, -S(O)-, -S(O)₂-, or -S(O)₂NR'-. In another embodiment, the substituent moieties R, R', R" and R''' are preferably independently selected from hydrogen, substituted or unsubstituted alkyl, substituted or unsubstituted cycloalkyl, substituted or unsubstituted heterocycloalkyl, substituted or unsubstituted aryl, and substituted or unsubstituted heteroaryl.

In another embodiment, the term "heteroatom" or "ring heteroatom" is meant to include oxygen (O), nitrogen (N), sulfur (S), phosphorus (P), and silicon (Si).

In another embodiment, the term "pharmaceutically acceptable salts" is meant to include salts of the active compounds which are prepared with relatively nontoxic acids or bases, depending on the particular substituent moieties found on the compounds described herein. In another embodiment, compounds of the present invention contain relatively acidic functionalities, base addition salts are obtained by contacting the neutral form of such compounds with a sufficient amount ofthe desired base, either neat or in a suitable inert solvent.

In another embodiment, pharmaceutically acceptable base addition salts include sodium, potassium, calcium, ammonium, organic amino, or magnesium salt, or a similar salt. In another embodiment, compounds of the present invention contain relatively basic functionalities, acid addition salts are obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. In another embodiment, also included are salts of amino acids such as arginate and the like, and salts of organic acids like glucuronic or galactunoric acids and the like (see, for example, Berge et al., "Pharmaceutical Salts", Journal of Pharmaceutical Science, 1977, 66, 1-19). In another embodiment, certain specific compounds of the present invention contain both basic and acidic functionalities that allow the compounds to be converted into either base or acid addition salts.

The neutral forms of the compounds are preferably regenerated by contacting the salt with a base or acid and isolating the parent compound in the conventional manner. In another embodiment, the parent form of the compound differs from the various salt forms in certain physical properties, such as solubility in polar solvents.

In another embodiment, the present invention provides compounds, which are in a prodrug form. In another embodiment, pro-drugs of the compounds described herein are those compounds that readily undergo chemical changes under physiological conditions to provide the compounds of the present invention. In another embodiment, pro-drugs are converted to the compounds of the present invention by chemical or biochemical methods in an ex vivo environment. In another embodiment, pro-drugs are slowly converted to the compounds of the present invention when placed in a transdermal patch reservoir with a suitable enzyme or chemical reagent.

In another embodiment, certain compounds of the present invention can exist in unsolvated forms as well as solvated forms, including hydrated forms. In another embodiment, the solvated forms are equivalent to unsolvated forms and are encompassed within the scope of the present invention. In another embodiment, compounds of the present invention may exist in multiple crystalline or amorphous forms. In another embodiment, all physical forms are equivalent for the uses contemplated by the present invention and are intended to be within the scope of the present invention.

In another embodiment, compounds of the present invention possess asymmetric carbon atoms (optical centers) or double bonds; the racemates, diastereomers, tautomers, geometric isomers and individual isomers are encompassed within the scope of the present invention. In another embodiment, compounds of the present invention do not include those which are known in the art to be too unstable to synthesize and/or isolate.

In another embodiment, the compounds of the present invention may also contain unnatural proportions of atomic isotopes at one or more of the atoms that constitute such compounds. In another embodiment, compounds may be radiolabeled with radioactive isotopes, such as for example tritium (³H), iodine-125 (¹²⁵I) or carbon-14 (¹⁴C). In another embodiment, all isotopic variations of the compounds of the present invention, whether radioactive or not, are encompassed within the scope of the present invention.

In another embodiment, where two groups are "optionally joined together to form a ring," the two groups are covalently bonded together with the atom or atoms to which the two groups are joined to form a substituted or unsubstituted aryl, a substituted or unsubstituted heteroaryl, a substituted or unsubstituted cycloalkyl, or a substituted or unsubstituted heterocycloalkyl ring.

In another embodiment, the terms "arylalkyl," "heteroarylalkyl," "cycloalkyl-alkyl," and "heterocycloalkyl-alkyl," as used herein, refers to an aryl, heteroaryl, cycloalkyl, and heterocycloalkyl, respectively, attached to the remainder of the molecule via an alkylene group. In another embodiment, an "arylalkyl," "heteroarylalkyl," "cycloalkyl-alkyl," or "heterocycloalkyl-alkyl" is substituted, one or more substituent moieties may be covalently bonded to the alkylene moiety and/or the aryl, heteroaryl, cycloalkyl and heterocycloalkyl moieties, respectively. In another embodiment, a "C1-C20" arylalkyl, heteroarylalkyl, cycloalkyl-alkyl, or heterocycloalkyl-alkyl, are moieties in which a C1-C20 alkylene links an aryl, heteroaryl, C4-C8 cycloalkyl, and 4 to 8 membered heterocycloalkyl, respectively, to the remainder of the molecule. In another embodiment, a "C1-C8" arylalkyl, heteroarylalkyl, cycloalkyl-alkyl, or heterocycloalkyl-alkyl, are moieties in which a C 1-C8 alkylene links an aryl, heteroaryl, C5-C7 cycloalkyl, and 5 to 7 membered heterocycloalkyl, respectively, to the remainder of the molecule.
In another embodiment, a "substituent group," as used herein, means a group selected from the following moieties:
(A) OH, NH₂, SH, CN, -CF₃, oxy, halogen, unsubstituted alkyl, unsubstituted heteroalkyl, unsubstituted cycloalkyl, unsubstituted heterocycloalkyl, unsubstituted aryl, unsubstituted heteroaryl, and
(B) alkyl, heteroalkyl, cycloalkyl, heterocycloalkyl, aryl, and heteroaryl, substituted with at least one substituent selected from:
   (i) oxy, -OH, -NH₂, SH, -CN, CF₃, halogen, unsubstituted alkyl, unsubstituted heteroalkyl, unsubstituted cycloalkyl, unsubstituted heterocycloalkyl, unsubstituted aryl, unsubstituted heteroaryl, and
   (ii) alkyl, heteroalkyl, cycloalkyl, heterocycloalkyl, aryl, and heteroaryl, substituted with at least one substituent selected from:
      (a) oxy, -OH, -NH₂, -SH, -CN, -CF₃, halogen, unsubstituted alkyl, unsubstituted heteroalkyl, unsubstituted cycloalkyl, unsubstituted heterocycloalkyl, unsubstituted aryl, unsubstituted heteroaryl, and
      (b) alkyl, heteroalkyl, cycloalkyl, heterocycloalkyl, aryl, or heteroaryl, substituted with at least one substituent selected from oxy, -OH, NH₂, -SH, -CN, CF₃, halogen, unsubstituted alkyl, unsubstituted heteroalkyl, unsubstituted cycloalkyl, unsubstituted heterocycloalkyl, unsubstituted aryl, and unsubstituted heteroaryl.

In another embodiment, a "size-limited substituent" or" size-limited substituent group," as used herein means a group selected from all of the substituents described above for a "substituent group,"
wherein each substituted or unsubstituted alkyl is a substituted or unsubstituted C1-C20 alkyl, each substituted or unsubstituted heteroalkyl is a substituted or unsubstituted 2 to 20 membered heteroalkyl, each substituted or unsubstituted cycloalkyl is a substituted or unsubstituted C4-C8 cycloalkyl, and each substituted or unsubstituted heterocycloalkyl is a substituted or unsubstituted 4 to 8 membered heterocycloalkyl.

In another embodiment, a "lower substituent" or "lower substituent group", as used herein means a group selected from all of the substituents described above for a "substituent group," wherein each substituted or unsubstituted alkyl is a substituted or unsubstituted C1-C8 alkyl, each substituted or unsubstituted heteroalkyl is a substituted or unsubstituted 2 to 8 membered heteroalkyl, each substituted or unsubstituted cycloalkyl is a substituted or unsubstituted C5-C7 cycloalkyl, and each substituted or unsubstituted heterocycloalkyl is a substituted or unsubstituted 5 to 7 membered heterocycloalkyl.

In another embodiment, the term "treating" refers to any indicia of success in the treatment or amelioration of an injury, pathology or condition, including any objective or subjective parameter such as abatement; remission; diminishing of symptoms or making the injury, pathology or condition more tolerable to the patient; slowing in the rate of degeneration or decline; making the final point of degeneration less debilitating; improving a patient's physical or mental well-being. In another embodiment, the treatment or amelioration of symptoms are based on objective or subjective parameters; including the results of a physical examination, neuropsychiatric exams, and/or a psychiatric evaluation. In another embodiment, the methods of the invention successfully treat a patient's delirium by decreasing the incidence of disturbances in consciousness or cognition.

In another embodiment, the term "higher alkyl" refers to those alkyl groups having at least six carbon atoms. In another embodiment, the term "lower alkyl" refers to those alkyl groups having from one to five carbon atoms.

In another embodiment, the present invention provides an antiproliferative, water soluble compound with high bioavailability having the general formula (I)

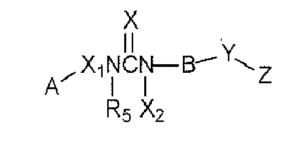

In another embodiment, A, B, and Z are independently substituted alkyl, cycloalkyl, aryl, heterocycle, or substituted phenyl fused with a saturated or unsaturated 4- to 6- membered rings optionally containing one to three heteroatoms independently selected from N, O, and S;
'X' is oxygen or sulfur;
'Y' is sulfur, sulfoxide or sulfone;
X₁ is CO or hydrogen;
R₅ is hydrogen, or alkyl or aryl group;
X₂ is hydrogen or SR₆, wherein R₆ is a substituted alkyl, cycloalkyl, aryl, heterocycle, or substituted phenyl fused with a saturated or unsaturated 4- to 6- membered rings optionally containing one to three heteroatoms independently selected from N, O, and S.

In another embodiment, the present invention is further defined by compounds having the general formulas (II) - (XXI): 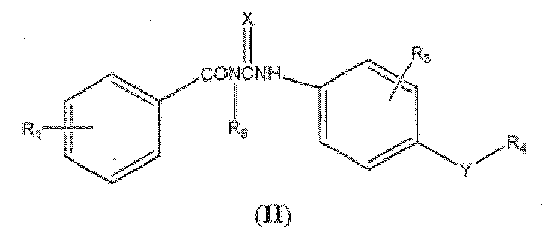 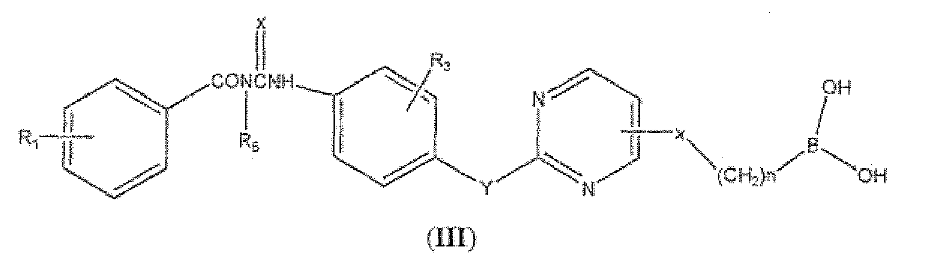 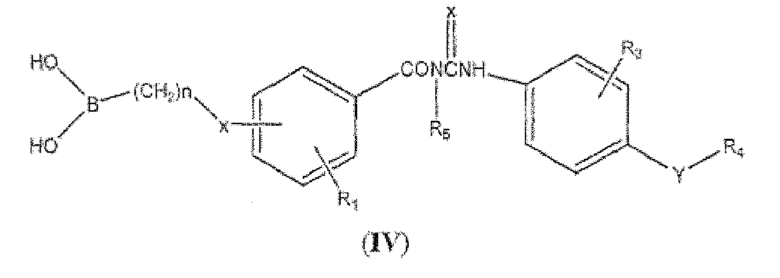 Where R₁ and R₃ are independently H, F, Cl, Br, I, CN, NO₂, NH₂, NHMe, NMe₂, CF₃, COOH, CONHOH, alkyl or aryl, group;
R₅ is alkyl or aryl group;
R₄ is substituted pyridine, pyrimidine, thiazole, pyrrazole, benzothiozole, indole, benzofuran or any heterocyclic ring;
'X' is oxygen or sulfur;
'Y' is sulfur, sulfoxide or sulfone; and
n is any integer. 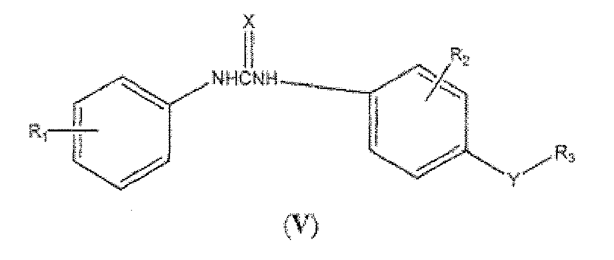 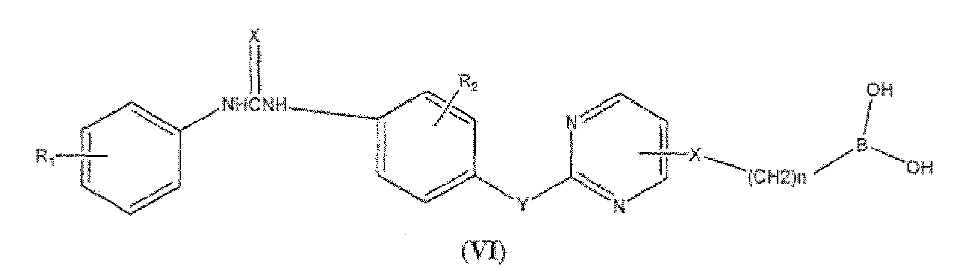 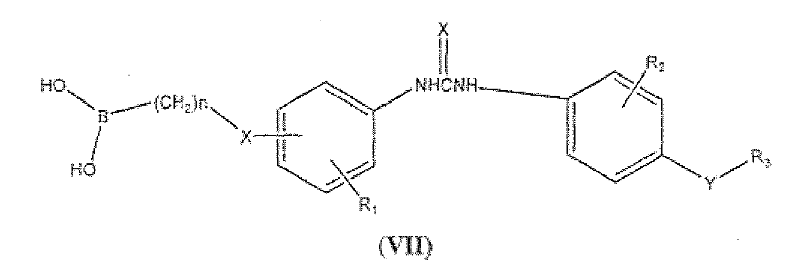 Where R₁ and R₂are independently H, F, Cl, Br, I, CN, NO₂, NH₂, NHMe, NMe₂, CF₃, COOH, CONHOH, alkyl or aryl group;
R₃ is substituted pyridine, pyrimidine, thiazole, pyrrazale, benzothiozole, indole, benzofuran or any heterocyclic ring;
'X' is oxygen or sulfur;
'Y' is sulfur, sulfoxide or sulfone; and
n is any integer. 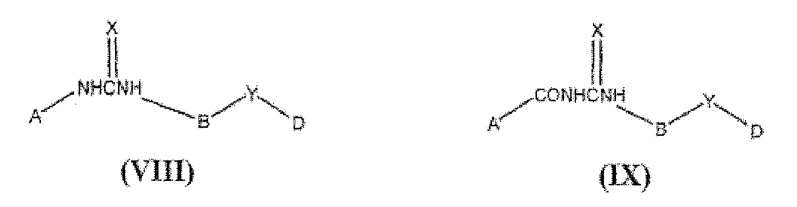 Where A, B, and D are independently substituted alkyl, cycloalkyl, aryl, heterocycle, or substituted phenyl fused with a saturated or unsaturated 4- to 6- membered rings optionally containing one to three heteroatoms independently selected from N, O, and S;
'X' is oxygen or sulfur; and
'Y' is sulfur, sulfoxide or sulfone.

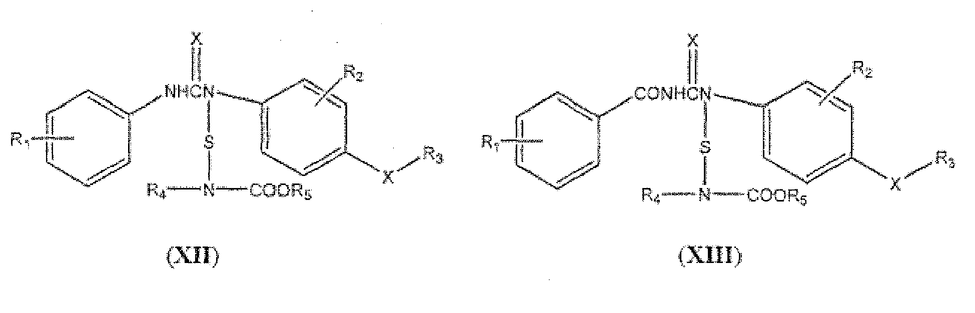

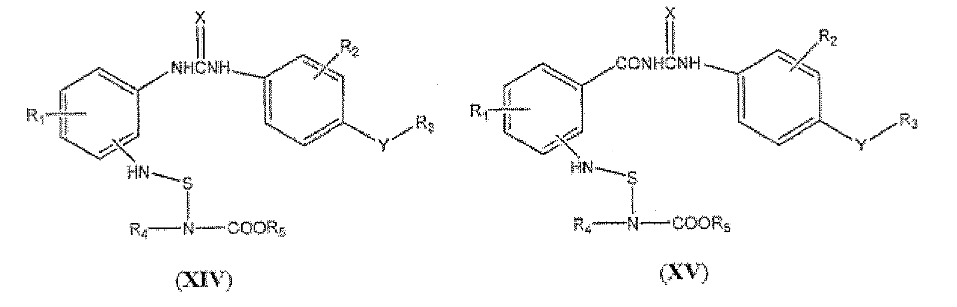

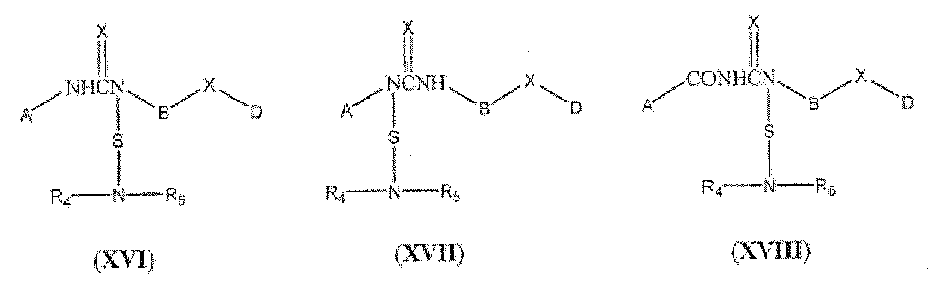

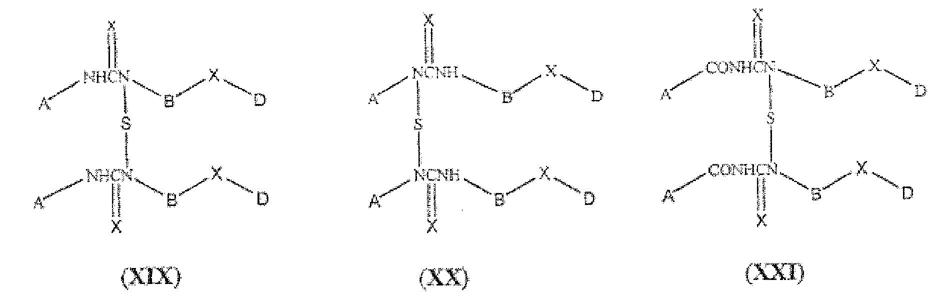

Where A, B, and D are independently substituted alkyl, cycloalkyl, aryl, heterocycle, or substituted phenyl fused with a saturated or unsaturated 4- to 6- membered rings optionally containing one to three heteroatoms independently selected from N, O, and S;
R₁ and R₂ are independently H, F, Cl, Br, I, CN, NO₂, NH₂, NHMe, NMe₂, CF₃, COOH, CONHOH, alkyl or arty group;
R₃ is substituted pyridine, pyrimidine, thiazole, pyrrazole, benzothiozole, indole, benzofuran or any heterocyclic ring;
R₄ and R₅ are independently substituted alkyl or aryl group; and
'X' is oxygen or sulfur.

In another embodiment, the compound of the present invention is compound XXX: 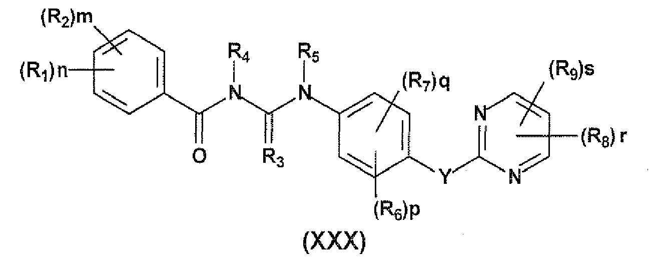 wherein R₁ and R₂ are independently H, NH₂, NO₂, Cl, Br, I, CN, NHMe, NMe₂, Me CF₃, COOH, CONHOH, alkyl or aryl;
R₆ and R₇ are independently H, NH₂, NO₂, F, Cl, Br, I, CN, NHMe, NMe₂, CF₃, COOH, CONHOH, alkyl or aryl;
Y is S, SO or SO₂;
R₃ is O or S;
R₄ and R₅ are independently, H or alkyl aryl or -SR₁₀, wherein R₁₀ is a substituted alkyl, cycloalkyl, aryl, heterocycle, or substituted phenyl fused with a saturated or unsaturated 4- to 6- membered rings optionally containing one to three heteroatoms independently selected from N, O, and S;
R₈ and R₉ are independently H, alkyl or halogen;
n is an integer between 1-5;
m is an integer between 0-4;
r is an integer between 1-3;
s is an integer between 0-2;
q is an integer between 1-4; and
p is an integer between 0-3.

In another embodiment, the compound ofthe present invention is compound XXX wherein R₄ is hydrogen. In another embodiment, the compound of the present invention is compound XXX wherein R₅ is hydrogen. In another embodiment, the compound of the present invention is compound XXX
wherein R₄ and R₅ are hydrogens. In another embodiment, the compound of the present invention is compound XXX wherein R₆ is methyl. In another embodiment, the compound of the present invention is compound XXX wherein R₈ is Br. In another embodiment, the compound of the present invention is compound XXX wherein R₁ is NH₂. In another embodiment, the compound of the present invention is compound XXX wherein R₁ is NO₂. In another embodiment, the compound of the present invention is compound XXX wherein R₈ is in the para position. In another embodiment, the compound of the present invention is compound XXX wherein R₁ is in the ortho position. In another embodiment, the compound of the present invention is compound XXX wherein s is 0. In another embodiment, the compound of the present invention is compound XXX wherein m is 0. In another embodiment, the compound of the present invention is compound XXX wherein s and m are 0.

In another embodiment, the compound of this invention is represented by formula 6n:

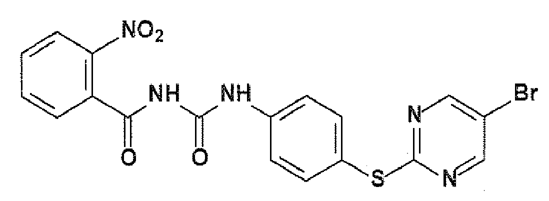

In another embodiment, the compound of this invention is represented by formula 7d:

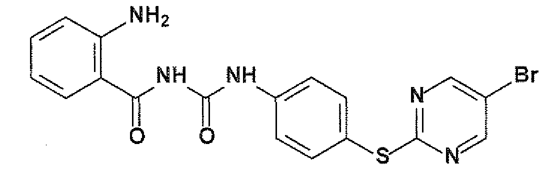

In another embodiment, the compound of the present invention is compound XXXI: 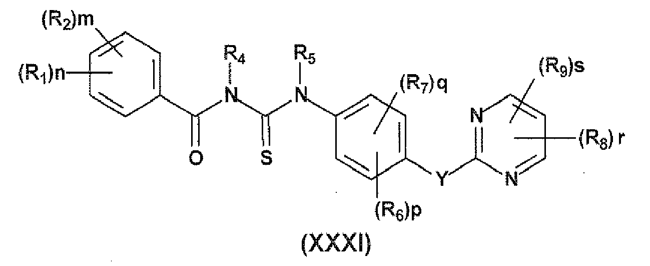 wherein R₁ and R₂, are independently H, NH₂, NO₂, F, Cl, Br, I, CN, NHMe, NMe₂, Me CF₃, COOH, CONHOH, alkyl or aryl;
R₆ and R₇ are independently H, NH₂, NO₂, F, Br, I, CN, NHMe, NMe₂, CF₃, COOH, CONHOH, alkyl or aryl;
Y is S, SO, SO₂ or O;
R₄ and R₅ are independently, H ,alkyl ,aryl or -SR₁₀, wherein R₁₀ is a substituted alkyl, cycloalkyl, aryl, heterocycle, or substituted phenyl fused with a saturated or unsaturated 4- to 6- membered rings optionally containing one to three heteroatoms independently selected from N, O, and S;
R₈ and R₉ are independently H or alkyl or halogen;
n is an integer between 1-5;
m is an integer between 0-4;
r is an integer between 1-3;
s is an integer between 0-2;
q is an integer between 1-4; and
p is an integer between 0-3.

In another embodiment, the compound of the present invention is compound XXXI wherein R₄ is hydrogen. In another embodiment, the compound of the present invention is compound XXXI
wherein R₅ is hydrogen. In another embodiment, the compound of the present invention is compound XXXI wherein R₄ and R₅ are hydrogens. In another embodiment, the compound of the present invention is compound XXXI wherein R₆ is methyl. In another embodiment, the compound of the present invention is compound XXXI wherein R₈ is Br. In another embodiment, the compound of the present invention is compound XXXI wherein R₁ is NH₂. In another embodiment, the compound of the present invention is compound XXXI wherein R₁ is NO₂. In another embodiment, the compound of the present invention is compound XXXI wherein R₈ is in the para position. In another embodiment, the compound of the present invention is compound XXXI wherein R₁ is in the ortho. In another embodiment, the compound of the present invention is compound XXXI wherein s is 0. In another embodiment, the compound of the present invention is compound XXXI wherein m is 0. In another embodiment, the compound of the present invention is compound XXXI wherein R₈ is in the paraposition and R₁ is in the ortho position and s and m are 0.

In another embodiment, the compound of the present invention is compound XXXII: 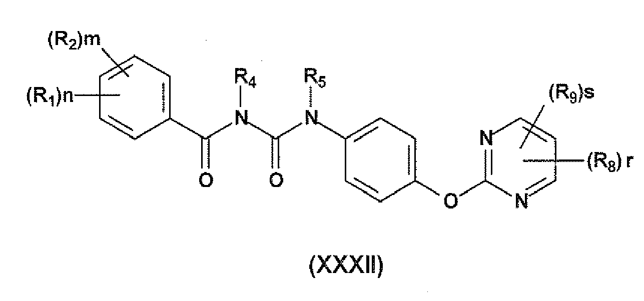 wherein, R₁ is NH₂, NHMe, NMe₂, or NO₂and R₂ are independently H, NH₂, NO₂, CN, NHMe, NMe₂, CF₃, COOH, CONHOH, alkyl or aryl;
R₄ and R₅ are independently, H alkyl, aryl or -SR₁₀, wherein R₁₀ is a substituted alkyl, cycloalkyl, aryl, heterocycle, or substituted phenyl fused with a saturated or unsaturated 4- to 6- membered rings optionally containing one to three heteroatoms independently selected from N, O, and S;
R₈ and R₉ are independently H, alkyl or halogen;
n is an integer between 1-5;
m is an integer between 0-4;
r is an integer between 1-3;
s is an integer between 0-2; and wherein if R₁ is NO₂ in the ortho position, n is 1, m is 0, s is 0, r is 1, R₄ and R₅ are H, than R₈ is not iodine, bromine or hydrogen in the para position.

In another embodiment, the compound of the present invention is compound XXXII wherein R₄ is hydrogen. In another embodiment, the compound of the present invention is compound XXXII
wherein R₅ is hydrogen. In another embodiment, the compound of the present invention is compound XXXII wherein R₄ and R₅ are hydrogens. In another embodiment, the compound of the present invention is compound XXXII wherein R₈ is Br. In another embodiment, the compound of the present invention is compound XXXII wherein R₁ is NH₂. In another embodiment, the compound of the present invention is compound XXXII wherein R₁ is NO₂. In another embodiment, the compound of the present invention is compound XXXII wherein R₈ is in the para position. In another embodiment, the compound of the present invention is compound XXXII wherein R₁ is in the ortho. In another embodiment, the compound of the present invention is compound XXXI wherein s is 0. In another embodiment, the compound of the present invention is compound XXXI wherein m is 0. In another embodiment, the compound of the present invention is compound XXXI wherein R₈ is in the para position and R₁ is in the ortho position and s and m are 0.

In another embodiment, the compounds of the present invention have the formulas provided in Table 1. These compounds were prepared according to the synthesis schemes that are described below.

**Table 1**

| **Compound** | **Structure** |
|---|---|
| **6a** | 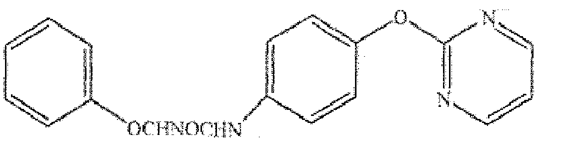 |
| **6b** | 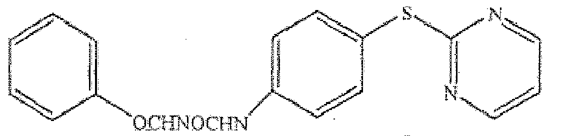 |
| **6c** | 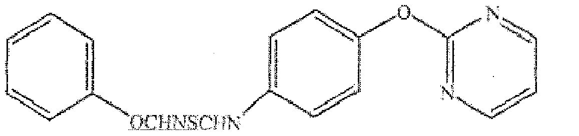 |
| **6d** | 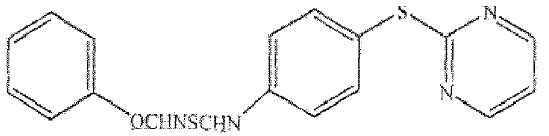 |
| **6e** | 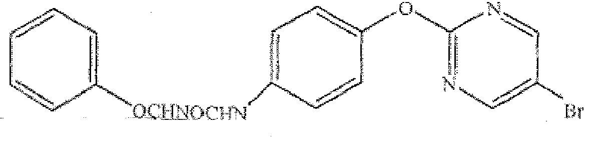 |
| **6f** | 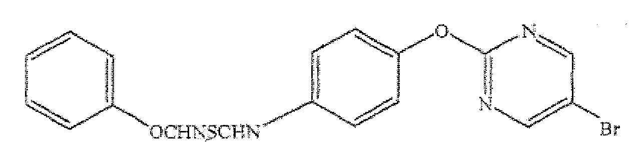 |
| **6g** | 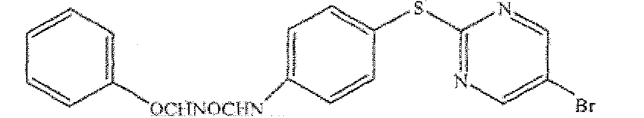 |
| **6h** | 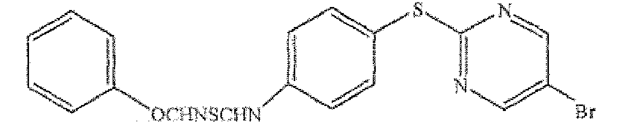 |
| **6i** | 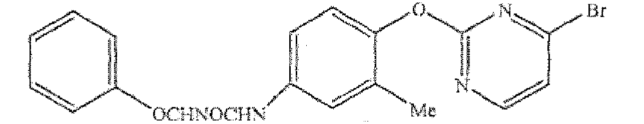 |
| **6j** | 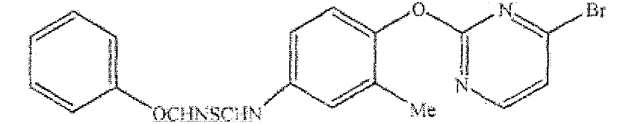 |
| **6k** | 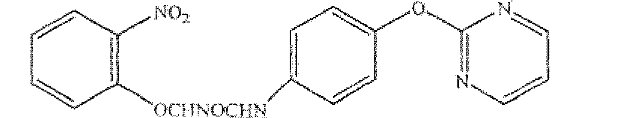 |
| **6l** | 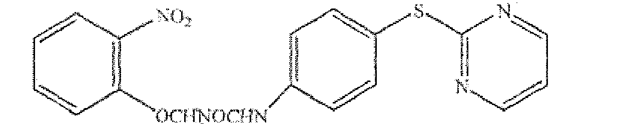 |
| **6m** | 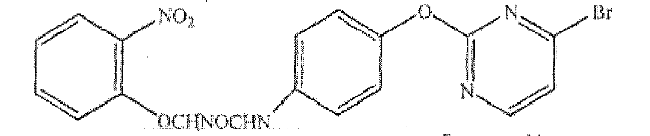 |
| **6n** | 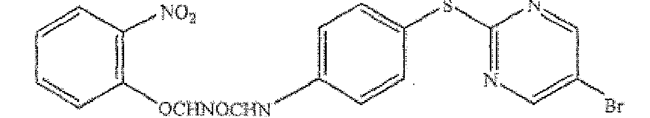 |
| **7a** | 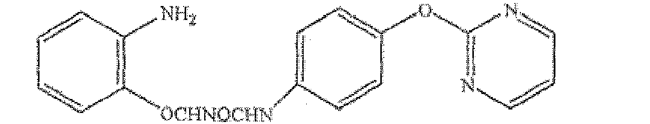 |
| **7b** | 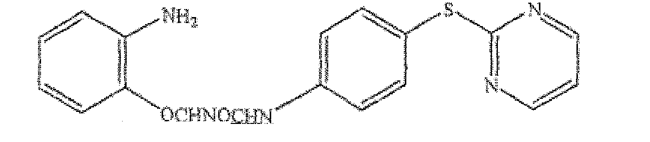 |
| **7c** | 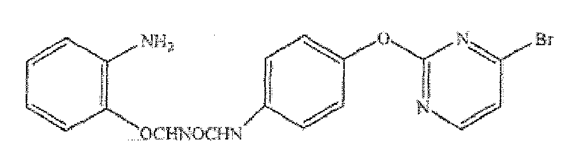 |
| **7d** | 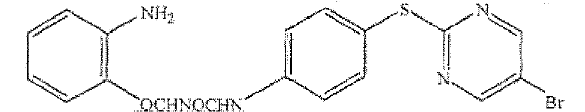 |
| **8a** | 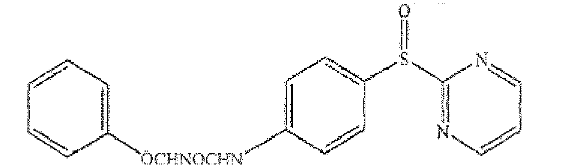 |
| **8b** | 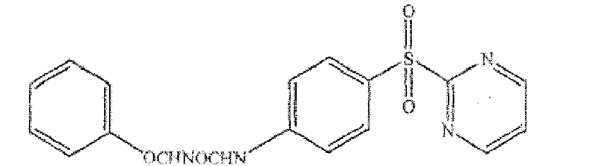 |
| **8c** | 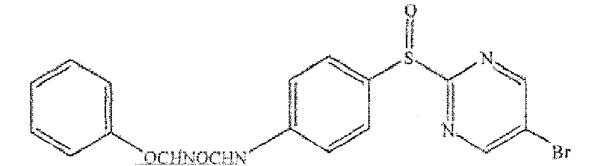 |
| **8d** | 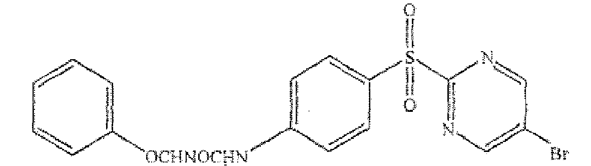 |
| **8e** | 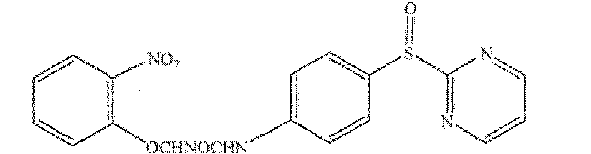 |
| **8f** | 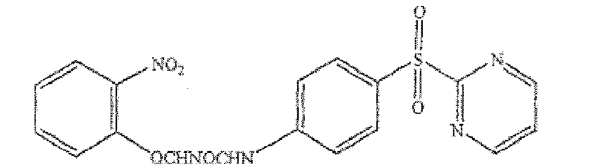 |
| **8g** | 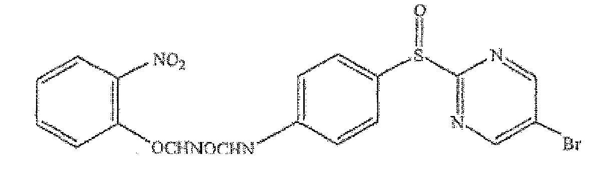 |
| **8h** | 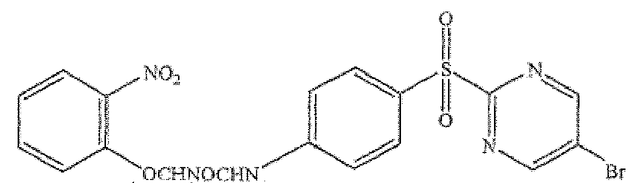 |
| **9** | 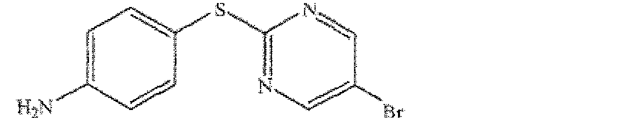 |
| **10** | 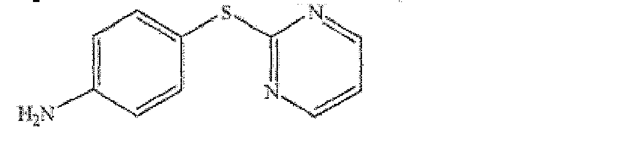 |

In another embodiment, the present invention provides pharmaceutical compositions. The pharmaceutical composition includes a pharmaceutically acceptable excipient and a compound having the formula (I - XXI and XXX-XXXII):

In another embodiment, the present invention is further defined by compounds having the general formulas (XXI) - (XXIII) where A, B, D, E and G are independently substituted alkyl, cycloalkyl, aryl, heterocycle, or substituted phenyl fused with a saturated or unsaturated 4- to 6-membered rings optionally containing one to three heteroatoms independently selected from N, O, and S; 'X' is oxygen or sulfur.

In another embodiment, the present invention provides an antiproliferative compound having the general formula (XXX). In another embodiment, the present invention provides a water soluble compound having the general formula (XXX). In another embodiment, the present invention provides an a high bioavailability having the general formula (XXX). In another embodiment, the present invention provides an antiproliferative, water soluble compound with high bioavailability having the general formula (XXX).

In another embodiment, the present invention provides an antiproliferative compound having the general formula (XXXI). In another embodiment, the present invention provides a water soluble compound having the general formula (XXXI). In another embodiment, the present invention provides a high bioavailability having the general formula (XXXI). In another embodiment, the present invention provides an antiproliferative, water soluble compound with high bioavailability having the general formula (XXXI).

In another embodiment, the present invention provides an antiproliferative compound having the general formula (XXXII). In another embodiment, the present invention provides a water soluble compound having the general formula (XXXII). In another embodiment, the present invention provides a high bioavailability having the general formula (XXXII). In another embodiment, the present invention provides an antiproliferative, water soluble compound with high bioavailability having the general formula (XXXII).

In another embodiment, the present invention provides pharmaceutical compositions comprising the compounds of the present invention. In another embodiment, the present invention provides that pharmaceutical compositions of the present invention are used in medicine by methods known to one of skill in the art. In another embodiment, the present invention provides that pharmaceutical compositions of the present invention are used in medicine by the methods of the invention for treating diseases such as cancer. In another embodiment, the present invention provides that pharmaceutical compositions described herein are typically used for chemotherapy of various human illnesses, such as but not limited to osteoporesis, psoriasis, kidney failure, and immunosuppressant disorders in a subject in need of such treatment.

In another embodiment, the present invention provides that the pharmaceutical composition includes from 1 to 3000 milligrams of a compound of Formula (I - XXI or XXX-XXXII). In another embodiment, the present invention provides that the pharmaceutical composition includes from 1 to 2500 milligrams of a compound of Formula (I - XXI or XXX-XXXII). In another embodiment, the present invention provides that the pharmaceutical composition includes from 200 to 3000 milligrams of a compound of Formula (I - XXI or XXX-XXXII). In another embodiment, the present invention provides that the pharmaceutical composition includes from 200 to 2000 milligrams of a compound of Formula (I - XXI or XXX-XXXII). In another embodiment, the present invention provides that the pharmaceutical composition includes from 1 to 1800 milligrams of a compound of Formula (I-XXI or XXX-XXXII). In another embodiment, the present invention provides that the pharmaceutical composition includes from 300 to 1800 milligrams of a compound of Formula (I - XXI or XXX-XXXII). In another embodiment, the present invention provides that the pharmaceutical composition includes from 500 to 1500 milligrams of a compound of Formula (I - XXI or XXX-XXXII). In another embodiment, the present invention provides that the pharmaceutical composition includes from 1000 to 3000 milligrams of a compound of Formula (I - XXI or XXX-XXXII). In another embodiment, the present invention provides that the pharmaceutical composition includes from 1500 to 2500 milligrams of a compound of Formula (I - XXI or XXX-XXXII).

In another embodiment, the present invention provides that the compounds of the present invention are prepared and administered in a wide variety of oral, parenteral and topical dosage forms. In another embodiment, the present invention provides that the oral preparations include tablets, pills, powder, dragees, capsules, liquids, lozenges, gels, syrups, slurries, suspensions, etc., suitable for ingestion by the patient. In another embodiment, the present invention provides that the compounds of the present invention can also be administered by injection, that is, intravenously, intramuscularly, intracutaneously, subcutaneously, intraduodenally, or intraperitoneally. In another embodiment, the present invention provides that the compounds described herein are administered by inhalation, for example, intranasally. In another embodiment, the present invention provides that the compounds of the present invention are administered transdermally. In another embodiment, the present invention provides that the compounds of this invention are administered by in intraocular, intravaginal, and intrarectal routes including suppositories, insufflation, powders and aerosol formulations (for examples of steroid inhalants, see Rohatagi. J. Clin. Pharmacol. 35:1187-1193, 1995; Tjwa, Ann. Allergy Asthma Immunol. 75:107-111, 1995). In another embodiment, the present invention provides that the pharmaceutical compositions described herein are adapted for oral administration. In another embodiment, the present invention provides that the pharmaceutical composition is in the form of a tablet. In another embodiment, the present invention provides that the pharmaceutical composition is in the form of a capsule. In another embodiment, the present invention provides that the pharmaceutical compositions include a pharmaceutically acceptable carrier or excipient and either a compound of Formula (I - XXI or XXX-XXXII), or a pharmaceutically acceptable salt of a compound of Formula (I - XXI or XXX-XXXII).

For preparing pharmaceutical compositions from the compounds of the present invention, pharmaceutically acceptable carriers are either solid or liquid. In another embodiment, the present invention provides that solid form preparations include powders, tablets, pills, capsules, cachets, suppositories, and dispersible granules. In another embodiment, the present invention provides that a solid carrier is one or more substances, which may also act as diluents, flavoring agents, binders, preservatives, tablet disintegrating agents, or an encapsulating material. In another embodiment, the present invention provides that details on techniques for formulation and administration are well described in the scientific and patent literature, see, e.g., the latest edition of Remington's Pharmaceutical Sciences, Maack Publishing Co, Easton PA ("Remington's").

In another embodiment, the present invention provides that in powders, the carrier is a finely divided solid, which is in a mixture with the finely divided active component. In another embodiment, the present invention provides that in tablets, the active component is mixed with the carrier having the necessary binding properties in suitable proportions and compacted in the shape and size desired.

In another embodiment, the present invention provides that the powders and tablets preferably contain from 5% or 10% to 70% of the active compound. In another embodiment, the present invention provides that the powders and tablets preferably contain from 2% to 90% of the active compound. In another embodiment, the present invention provides that the powders and tablets preferably contain from 2% to 50% of the active compound. In another embodiment, the present invention provides that the powders and tablets preferably contain from 5% to 90% of the active compound. In another embodiment, the present invention provides that the powders and tablets preferably contain from 5% to 30% of the active compound. In another embodiment, the present invention provides that the powders and tablets preferably contain from 10% to 50% of the active compound. In another embodiment, the present invention provides that the powders and tablets preferably contain from 30% to 70% of the active compound. In another embodiment, the present invention provides that the powders and tablets preferably contain from 50% to 90% of the active compound. In another embodiment, the present invention provides that the powders and tablets preferably contain from 40% to 60% of the active compound. In another embodiment, the present invention provides that the powders and tablets preferably contain from 2% to 20% of the active compound.

In another embodiment, the present invention provides that suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. In another embodiment, the present invention provides that the term "preparation" is intended to include the formulation of the active compound with encapsulating material as a carrier providing a capsule in which the active component with or without other carriers, is surrounded by a carrier, which is thus in association with it. Similarly, cachets and lozenges are included. In another embodiment, the present invention provides that the tablets, powders, capsules, pills, cachets, and lozenges are used as solid dosage forms suitable for oral administration.

In another embodiment, the present invention provides that suitable solid excipients are carbohydrate or protein fillers include, but are not limited to sugars, including lactose, sucrose, mannitol, or sorbitol; starch from corn, wheat, rice, potato, or other plants; cellulose such as methyl cellulose, hydroxypropylmethyl-cellulose, or sodium carboxymethylcellulose; and gums including Arabic gum and tragacanth; as well as proteins such as gelatin and collagen. In another embodiment, the present invention provides that the composition further comprises disintegrating or solubilizing agents, such as but not limited to the cross-linked polyvinyl pyrrolidone, agar, alginic acid, or a salt thereof, such as sodium alginate.

In another embodiment, the present invention provides that dragee cores comprise suitable coatings such as but not limited to concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. In another embodiment, the present invention provides that dyestuffs or pigments are added to the tablets or dragee coatings for product identification or to characterize the quantity of active compound (i.e., dosage). In another embodiment, pharmaceutical preparations of the invention are used orally using, for example, push-fit capsules made of gelatin, as well as soft, sealed capsules made of gelatin and a coating such as glycerol or sorbitol. In another embodiment, the present invention provides that push-fit capsules contain the compounds of this invention mixed with a filler or binders such as lactose or starches, lubricants such as talc or magnesium stearate, and, optionally, stabilizers. In another embodiment, the present invention provides that in soft capsules, the compounds of this invention compounds are dissolved or suspended in suitable liquids, such as fatty oils, liquid paraffin, or liquid polyethylene glycol with or without stabilizers.

In another embodiment, for preparing suppositories, a low melting wax, such as a mixture of fatty acid glycerides or cocoa butter, is first melted and the active component is dispersed homogeneously therein, as by stirring. In another embodiment, the present invention provides that the molten homogeneous mixture is then poured into convenient sized molds, allowed to cool, and thereby to solidify.

In another embodiment, the present invention provides that liquid form preparations include solutions, suspensions, and emulsions, for example, water or water/propylene glycol solutions. In another embodiment, the present invention provides that for parenteral injection, liquid preparations are formulated in solution in aqueous polyethylene glycol solution.

In another embodiment, the present invention provides that aqueous solutions suitable for oral use are prepared by dissolving the active component in water and adding suitable colorants, flavors, stabilizers, and thickening agents as desired. In another embodiment, the present invention provides that aqueous suspensions suitable for oral use are made by dispersing the finely divided active component in water with viscous maternal such as natural or synthetic gums, resins, methylcellulose, sodium carboxymethylcellulose, hydroxypropylmethylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethylene oxycetanol), a condensation product of ethylene oxide with a partial ester derived from a fatty acid and a hexitol (e.g., polyoxyethylene sorbitol mono-oleate), or a condensation product of ethylene oxide with a partial ester derived from fatty acid and a hexitol anhydride (e.g., polyoxyethylene sorbitan mono-oleate). In another embodiment, the present invention provides that the aqueous suspension contains one or more preservatives such as ethyl or n-propyl p-hydroxybenzoate, one or more coloring agents, one or more flavoring agents and one or more sweetening agents, such as sucrose, aspartame or saccharin. Formulations are adjusted for osmolarity.

In another embodiment, the present invention provides that also included are solid form preparations, which are intended to be converted, shortly before use, to liquid form preparations for oral administration. In another embodiment, the present invention provides that such liquid forums include solutions, suspensions, and emulsions. In another embodiment, the present invention provides that these preparations contain, in addition to the active component, colorants, flavors, stabilizers, buffers, artificial and natural sweeteners, dispersants, thickeners, solubilizing agents, and the like.

In another embodiment, the present invention provides that oil suspensions are formulated by suspending a compound of this invention in a vegetable oil, such as arachis oil, olive oil, sesame oil or coconut oil, or in a mineral oil such as liquid paraffin; or a mixture of these. In another embodiment, the present invention provides that oil suspensions contain a thickening agent, such as beeswax, hard paraffin or cetyl alcohol. In another embodiment, the present invention provides that sweetening agents are added to provide a palatable oral preparation, such as glycerol, sorbitol or sucrose. In another embodiment, the present invention provides that these formulations are preserved by the addition of an antioxidant such as ascorbic acid. As an example of an injectable oil vehicle, see Minto, J. Pharmacol. Exp. Ther., 281:93-102 (1997). In another embodiment, the present invention provides that the pharmaceutical formulations of the invention can also be in the form of oil-in-water emulsions. In another embodiment, the present invention provides that the oily phase is a vegetable oil or a mineral oil, described above, or a mixture of these.

In another embodiment, the present invention provides that suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. In another embodiment, the present invention provides that the emulsion also contains sweetening agents and flavoring agents, as in the formulation of syrups and elixirs. Such formulations can also contain a demulcent, a preservative, or a coloring agent.

In another embodiment, the present invention provides that the compounds of this are delivered by transdermally, by a topical route, formulated as applicator sticks, solutions, suspensions, emulsions, gels, creams, ointments, pastes, jellies, paints, powders, and aerosols.

In another embodiment, the present invention provides that the compounds ofthis invention are delivered as microspheres for slow release in the body. For example, microspheres are administered via intradermal injection of drug-containing microspheres, which slowly release subcutaneously (see Rao, J. Biomater Sci. Polym. Ed., 7:623-645 (1995); as biodegradable and injectable gel formulations (see, e.g., Gao Pharm. Res., 12:857-863 (1995); or, as microspheres for oral administration (see, e.g., Eyles, J. Pharm. Pharmacol., 49:669-674 (1997). Both transdermal and intradermal routes afford constant delivery for weeks or months.

In another embodiment, the present invention provides that the compounds of this invention are provided as a salt and are formed with many acids, including but not limited to hydrochloric, sulfuric, acetic, lactic, tartaric, maleic, succinic, etc. In another embodiment, the present invention provides that salts tend to be more soluble in aqueous or other protonic solvents that are the corresponding free base forms. In another embodiment, the present invention provides that In the preparation is a lyophilized powder in 1 mM-50 mM histidine, 0.1%-2% sucrose, 2%-7% mannitol at a pH range of 4.5 to 5.5, that is combined with buffer prior to use.

In another embodiment, the compounds of this invention having the formulations of the invention are useful for parenteral administration, such as intravenous (IV) administration or administration into a body cavity or lumen of an organ. In another embodiment, the present invention provides that formulations for administration will commonly comprise a solution of the compounds of this invention dissolved in a pharmaceutically acceptable carrier. In another embodiment, the present invention provides that among the acceptable vehicles and solvents that are employed are water and Ringer's solution, an isotonic sodium chloride. In another embodiment, the present invention provides that sterile fixed oils can conventionally be employed as a solvent or suspending medium. For this purpose any bland fixed oil is employed including synthetic mono- or diglycerides. In another embodiment, the present invention provides that fatty acids such as oleic acid can likewise be used in the preparation of injectables. In another embodiment, the present invention provides that solutions are sterile and generally free of undesirable matter. In another embodiment, the present invention provides that these formulations are sterilized by conventional, well known sterilization techniques. In another embodiment, the present invention provides that formulations contain pharmaceutically acceptable auxiliary substances as required to approximate physiological conditions such as pH adjusting and buffering agents, toxicity adjusting agents, e.g., sodium acetate, sodium chloride, potassium chloride, calcium chloride, sodium lactate and the like. In another embodiment, the present invention provides that the concentration of the compounds of this invention in these formulations can vary widely, and will be selected primarily based on fluid volumes, viscosities, body weight, and the like, in accordance with the particular mode of administration selected and the patient's needs.

In another embodiment, the present invention provides that for IV administration, the formulation can is a sterile injectable preparation, such as a sterile injectable aqueous or oleaginous suspension. In another embodiment, the present invention provides that this suspension is formulated according to the known art using those suitable dispersing or wetting agents and suspending agents. In another embodiment, the present invention provides that the sterile injectable preparation can also be a sterile injectable solution or suspension in a nontoxic parenterally-acceptable diluent or solvent, such as a solution of 1, 3-butanediol.

In another embodiment, the present invention provides that formulations having the compounds of this invention are delivered by the use of liposomes which fuse with the cellular membrane or are endocytosed, i.e., by employing ligands attached to the liposome, or attached directly to the oligonucleotide, that bind to surface membrane protein receptors of the cell resulting in endocytosis. In another embodiment, the present invention provides that using liposomes, particularly where the liposome surface carries ligands specific for target cells, or are otherwise preferentially directed to a specific organ, one can focus the delivery of the compounds of this invention into the target cells in vivo. (See, e.g., Al-Muhammed, J. Microencapsul., 13:293-306, 1996; Chonn, Curr. Opin. Biotechnol., 6:698-708 (1995); Ostro, Am. J. Hosp. Pharm., 46:1576-1587 (1989).

In another embodiment, the present invention provides that the pharmaceutical preparation is preferably in unit dosage form. In another embodiment, the present invention provides that in such fonn the preparation is subdivided into unit doses containing appropriate quantities of the active component. In another embodiment, the present invention provides that the unit dosage form is a packaged preparation, the package containing discrete quantities of preparation, such as packaged tablets, capsules, and powders in vials or ampoules. In another embodiment, the present invention provides that the unit dosage form is a capsule, tablet, cachet, or lozenge itself, or it can be the appropriate number of any of these in packaged form.

In another embodiment, the present invention provides that the quantity of active component (compound of the present invention) in a unit dose preparation is varied or adjusted from 0.05 mg to 10000 mg, more typically 1.0 mg to 1000 mg, most typically 10 mg to 500 mg, according to the particular application and the potency of the active component. In another embodiment, the present invention provides that the quantity of active component (compound of the present invention) in a unit dose preparation is varied or adjusted from 0.05 mg to 1000 mg. In another embodiment, the present invention provides that the quantity of active component (compound of the present invention) in a unit dose preparation is varied or adjusted from 1 mg to 1000 mg. In another embodiment, the present invention provides that the quantity of active component (compound of the present invention) in a unit dose preparation is varied or adjusted from 5 mg to 800 mg. In another embodiment, the present invention provides that the quantity of active component (compound of the present invention) in a unit dose preparation is varied or adjusted from 50 mg to 1000 mg. In another embodiment, the present invention provides that the quantity of active component (compound of the present invention) in a unit dose preparation is varied or adjusted from 10 mg to 200 mg. In another embodiment, the present invention provides that the quantity of active component (compound of the present invention) in a unit dose preparation is varied or adjusted from 50 mg to 500 mg. In another embodiment, the present invention provides that the quantity of active component (compound of the present invention) in a unit dose preparation is varied or adjusted from 500 mg to 4000 mg. In another embodiment, the present invention provides that the quantity of active component (compound of the present invention) in a unit dose preparation is varied or adjusted from 1000 mg to 3000 mg. In another embodiment, the present invention provides that the quantity of active component (compound of the present invention) in a unit dose preparation is varied or adjusted from 1 mg to 100 mg. In another embodiment, the present invention provides that the quantity of active component (compound of the present invention) in a unit dose preparation is varied or adjusted from 10 mg to 100 mg. In another embodiment, the present invention provides that the quantity of active component (compound ofthe present invention) in a unit dose preparation is varied or adjusted from 100 mg to 800 mg. In another embodiment, the present invention provides that the quantity of active component (compound of the present invention) in a unit dose preparation is varied or adjusted from 500 mg to 1000 mg. In another embodiment, the present invention provides that the composition can, if desired, also contain other compatible therapeutic agents.

In another embodiment, the present invention provides a method for the treatment of a disorder or condition which requires chemotherapy. In another embodiment, the present invention provides that a subject in need of such treatment is administered an effective amount of a compound having one of the formulae provided above. In another embodiment, the present invention provides that the amount of a compound of the present invention is effective in treating the illness that the patient is afflicted with.

In another embodiment, the present invention provides a variety of disease states are capable of being treated with chemotherapy. In another embodiment, the present invention provides that exemplary disease states include, but are not limited to cancer, kidney failure, osteoporesis, psoriasis, kidney failure, and immunosuppressant disorders. In another embodiment, the present invention provides that the methods of treatment includes administering to a patient in need of such treatment, a therapeutically effective amount of a compound according to Formula (I - XXI or XXX-XXXII), or a pharmaceutically acceptable salt thereof. In another embodiment, the present invention provides that the methods of treatment includes administering to a patient in need of such treatment, a therapeutically effective amount of a combination of compounds of formulas I - XXI or XXX-XXXII, or a pharmaceutically acceptable salts thereof. In another embodiment, the present invention provides that the methods of treatment include administering to a patient in need of such treatment, a therapeutically effective amount of a compound of formulas I - XXI or XXX-XXXII and an additional compound suited to treat a particular disease as known to one of skill in the art.

In another embodiment, the present invention provides a method of treating a disorder or condition through chemotherapy, the method including administering to a subject in need of such treatment, an effective amount of a compound of the present invention, such as a compound of Formula (I - XXI or XXX-XXXII).

In another embodiment, the present invention provides that the amount of the compounds of this invention adequate to treat a disease is defined as a "therapeutically effective dose". In another embodiment, the present invention provides the dosage schedule and amounts effective for this use, i.e., the "dosing regimen," will depend upon a variety of factors, including the stage of the disease or condition, the severity of the disease or condition, the general state of the patient's health, the patient's physical status, age and the like. In another embodiment, the present invention provides that in calculating the dosage regimen for a patient, the mode of administration also is taken into consideration.

In another embodiment, the present invention provides that the dosage regimen also takes into consideration pharmacokinetics parameters well known in the art, i.e., the rate of absorption, bioavailability, metabolism, clearance, and the like (see, e.g., Hidalgo-Aragones, J. Steroid Biochem. Mol. Biol., 58:611-617 (1996); Groning, Pharmazie, 51:337-341 (1996); Fotherby, Contraception, 54:59-69 (1996); Johnson, J. Charm. Sci., 84:1144-1146 (1995); Rohatagi, Pharmazie, 50:610-613 (1995); Brophy, Eur. J. Clin. Pharmacol., 24: 103-108 (1983); the latest Remington's, supra). In another embodiment, the present invention provides the state of the art allows the clinician to determine the dosage regimen for each individual patient, and disease or condition treated.

In another embodiment, the present invention provides that a single or multiple administrations of formulations having the compounds of this invention are administered depending on the dosage and frequency as required and tolerated by the patient. In another embodiment, the present invention provides that the formulations should provide a sufficient quantity of active agent to effectively treat the disease state.

In another embodiment, the present invention provides that the pharmaceutical formulations for oral administration of the compounds of this invention is in a daily amount of between about 0.5 to about 20 mg per kilogram of body weight per day. In an alternative embodiment, dosages are from about 1 mg to about 4 mg per kg of body weight per patient per day are used. In another embodiment, the present invention provides that the pharmaceutical formulations for oral administration of the compounds of this invention is in a daily amount of between about 0.5 to about 10 mg per kilogram of body weight per day. In another embodiment, the present invention provides that the pharmaceutical formulations for oral administration of the compounds of this invention is in a daily amount of between about 10 to about 200 mg per kilogram of body weight per day. In another embodiment, the present invention provides that the pharmaceutical formulations for oral administration ofthe compounds of this invention is in a daily amount of between about 0.5 to about 2 mg per kilogram of body weight per day. In another embodiment, the present invention provides that the pharmaceutical formulations for oral administration of the compounds of this invention is in a daily amount of between about 1 to about 20 mg per kilogram of body weight per day. In another embodiment, the present invention provides that the pharmaceutical formulations for oral administration of the compounds of this invention is in a daily amount of between about 5 to about 15 mg per kilogram of body weight per day. In another embodiment, the present invention provides that the pharmaceutical formulations for oral administration of the compounds of this invention is in a daily amount of between about 0.01 to about 2 mg per kilogram of body weight per day. In another embodiment, the present invention provides that the pharmaceutical formulations for oral administration of the compounds of this invention is in a daily amount of between about 0.5 to about 20 mg per kilogram of body weight per day. In another embodiment, the present invention provides that lower dosages are used, particularly when the drug is administered to an anatomically secluded site, such as the cerebral spinal fluid (CSF) space, in contrast to administration orally, into the blood stream, into a body cavity or into a lumen of an organ. Substantially higher dosages are used in topical administration.

In another embodiment, the present invention provides that actual methods for preparing parenterally administrable formulations having the compounds of this invention will be known or apparent to those skilled in the art and are described in more detail in such publications as Remington's, supra. See also Nieman, In "Receptor Mediated Antisteroid Action," Agarwal, et al., eds., De Gruyter, New York (1987).

In another embodiment, the present invention provides that after a pharmaceutical composition including a compound of the invention has been formulated in an acceptable carrier, it is placed in an appropriate container and labeled for treatment of an indicated condition. In another embodiment, the present invention provides that for administration of the compounds of this invention, such labeling would include, e.g., instructions concerning the amount, frequency and method of administration. In another embodiment, the present invention provides that for a kit for the treatment of cancer or kidney failure in a human which includes the compounds of this invention and instructional material teaching the indications, dosage and schedule of administration of the compounds of this invention.

In another embodiment, the methods of the present invention provide inhibiting the proliferation of a cancerous cell in vitro. In another embodiment, the methods of the present invention provide inhibiting the proliferation of a cancerous cell ex vivo. In another embodiment, the methods of the present invention provide inhibiting the proliferation of a cancerous cell in vivo. In another embodiment, the methods of the present invention provide treating a subject affected with cancer with a compound of the invention. In another embodiment, the invention the subject affected with cancer suffers from tumors and/or cell metastasis. In another embodiment, the cancer to be treated with the compounds of the present invention is: adrenocortical carcinoma, anal cancer, bladder cancer, brain tumour, brain stem glioma, brain tumor, cerebellar astrocytoma, cerebral astrocytoma, ependymoma, medulloblastoma, supratentorial primitive neuroectodermal, pineal tumors, hypothalamic glioma, breast cancer, carcinoid tumor, carcinoma, cervical cancer, colon cancer, endometrial cancer, esophageal cancer, extrahepatic bile duct cancer, wings family of tumors (pnet), extracranial germ cell tumor, eye cancer, intraocular melanoma, gallbladder cancer, gastric cancer, germ cell tumor, extragonadal, gestational trophoblastic tumor, head and neck cancer, hypopharyngeal cancer, islet cell carcinoma, laryngeal cancer, leukemia, acute lymphoblastic, leukemia, oral cavity cancer, liver cancer, lung cancer, small cell, lymphoma, AIDS-related, lymphoma, central nervous system (primary), lymphoma, cutaneous T-cell, lymphoma, hodgkin's disease, non-hodgkin's disease, malignant mesothelioma, melanoma, merkel cell carcinoma, metasatic squamous carcinoma, multiple myeloma, plasma cell neoplasms, mycosis fungoides, myelodysplastic syndrome, myeloproliferative disorders, nasopharyngeal cancer, neuroblastoma, oropharyngeal cancer, osteosarcoma, ovarian epithelial cancer, ovarian germ cell tumor, ovarian low malignant potential tumor, pancreatic cancer, exocrine, pancreatic cancer, islet cell carcinoma, paranasal sinus and nasal cavity cancer, parathyroid cancer, penile cancer, pheochromocytoma cancer, pituitary cancer, plasma cell neoplasm, prostate cancer, rhabdomyosarcoma, rectal cancer, renal cell cancer, salivary gland cancer, sezary syndrome, skin cancer, cutaneous T-cell lymphoma, skin cancer, Kaposi's sarcoma, skin cancer, melanoma, small intestine cancer, soft tissue sarcoma, soft tissue sarcoma, testicular cancer, thymoma, malignant, thyroid cancer, urethral cancer, uterine cancer, sarcoma, unusual cancer of childhood, vaginal cancer, vulvar cancer, or wilms' tumor.

The invention is characterized by the following items:
1. A compound represented by the structure of formula XXX: 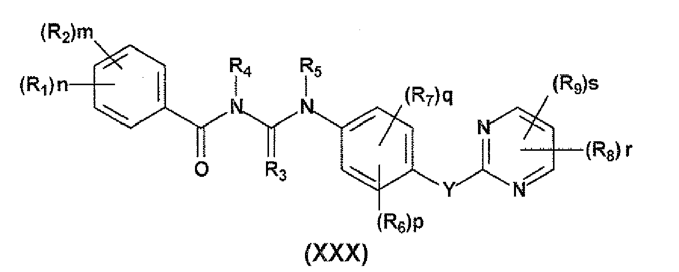 wherein R₁, and R₂ are independently H, NH₂, NO₂, Cl, Br, I, CN, NHMe, NMe₂, Me, CF₃, COOH, CONHOH, alkyl or aryl;
   R₆ and R₇ are independently H, NH₂, NO₂, F, Cl, Br, I, CN, NHMe, NMe₂, CF₃, COOH, CONHOH, alkyl or aryl;
   Y is S, SO or SO₂;
   R₃ is O or S;
   R₄ and R₅ are independently, H, alkyl ,aryl or -SR₁₀, wherein R₁₀ is a substituted alkyl, cycloalkyl, aryl, heterocycle, or substituted phenyl fused with a saturated or unsaturated 4- to 6- membered rings optionally containing one to three heteroatoms independently selected from N, O, and S;
   R₈ and R₉ are independently H, alkyl or halogen;
   n is an integer between 1-5;
   m is an integer between 0-4;
   r is an integer between 1-3;
   s is an integer between 0-2;
   q is an integer between 1-4; and
   p is an integer between 0-3.
2. The compound of item 1, wherein R₄ and R₅ are hydrogens.
3. The compound of item 1, wherein R₆ is methyl.
4. The compound of item 1, wherein R₈ is Br.
5. The compound of item 1, wherein R₁ is NH₂.
6. The compound of item 1, wherein R₁ is NO₂.
7. The compound of item 1, wherein R₈ is in the para position.
8. The compound of item 1, wherein R1 is in the ortho position.
9. The compound of item 1, wherein s and m are 0.
10. A composition comprising the compound of item 1, or its isomer, pharmaceutically acceptable salt, *N*-oxide, hydrate or any combination thereof; and a suitable carrier or diluent.
11. A method of treating a subject with a pancreatic cancer, said method comprising the step of administering to said subject a compound of item 1, or its pharmaceutical product, hydrate, pharmaceutically acceptable salt or any combination thereof, thereby treating a subject with a pancreatic cancer
12. A method of treating a subject with a prostate cancer, said method comprising the step of administering to said subject a compound of item 1, or its pharmaceutical product, hydrate, pharmaceutically acceptable salt or any combination thereof, thereby treating a subject with a pancreatic cancer
13. A method of inhibiting a replication of a pancreatic cancel cell, said method comprising the step of administering to said subject a compound of item 1, or its pharmaceutical product, hydrate, pharmaceutically acceptable salt or any combination thereof, thereby inhibiting a replication of a pancreatic cancer cell.
14. A composition comprising the compound of item 1, or its isomer, pharmaceutically acceptable salt, N-oxide, hydrate or any combination thereof; and a suitable carrier or diluent.
15. A method of treating a subject with a pancreatic cancer, said method comprising the step of administering to said subject a compound of item 1, or its pharmaceutical product, hydrate, pharmaceutically acceptable salt or any combination thereof, thereby treating a subject with a pancreatic cancer
16. A method of treating a subject with a prostate cancer, said method comprising the step of administering to said subject a compound of item 1, or its pharmaceutical product, hydrate, pharmaceutically acceptable salt or any combination thereof, thereby treating a subject with a pancreatic cancer
17. A method of inhibiting a replication of a pancreatic cancer cell, said method comprising the step of administering to said subject a compound of item 1, or its pharmaceutical product, hydrate, pharmaceutically acceptable salt or any combination thereof, thereby inhibiting a replication of a pancreatic cancer cell.
18. A method of inhibiting a replication of a prostate cancer cell, said method comprising the step of administering to said subject a compound of item 1, or its pharmaceutical product, hydrate, pharmaceutically acceptable salt or any combination thereof, thereby inhibiting a replication of a pancreatic cancer cell.
19. A compound represented by the structure of formula XXXI: 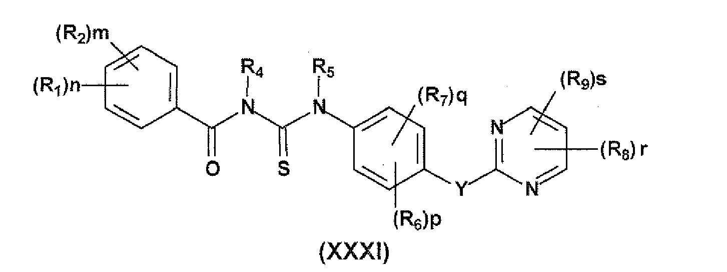 wherein R₁ and R₂, are independently H, NH₂, NO₂, F, Cl, Br, I, CN, NHMe, NMe₂, Me, CF₃, COOH, CONHOH, alkyl or aryl;
   R₆ and R₇ are independently H, NH₂, NO₂, F, Br, I, CN, NHMe, NMe₂, CF₃, COOH, CONHOH, alkyl or aryl;
   Y is S, SO, SO₂ or O;
   R₄ and R₅ are independently, H, alkyl ,aryl or -SR₁₀, wherein R₁₀ is a substituted alkyl, cycloalkyl, aryl, heterocycle, or substituted phenyl fused with a saturated or unsaturated 4- to 6-membered rings optionally containing one to three heteroatoms independently selected from N,
   O, and S;
   R₈ and R₉ are independently H or alkyl or halogen;
   n is an integer between 1-5;
   m is an integer between 0-4;
   r is an integer between 1-3;
   s is an integer between 0-2;
   q is an integer between 1-4 and
   p is an integer between 0-3.
20. The compound of item 19, wherein R₄ and R₅ are hydrogens.
21. The compound of item 19, wherein R₆ is methyl.
22. The compound of item 19, wherein R₈ is Br.
23. The compound of item 19, wherein R₁ is NH₂.
24. The compound of item 19, wherein R₁ is NO₂.
25. The compound of item 19, wherein R₈ is in the para position
26. The compound of item 19, wherein R₁ is in the ortho position.
27. The compound of item 19, wherein s and m are 0.
28. A composition comprising the compound of item 19, or its isomer, pharmaceutically acceptable salt, *N*-oxide, hydrate or any combination thereof; and a suitable carrier or diluent.
29. A method of treating a subject with a pancreatic cancer, said method comprising the step of administering to said subject a compound of item 19, or its pharmaceutical product, hydrate, pharmaceutically acceptable salt or any combination thereof, thereby treating a subject with a pancreatic cancer
30. A method of treating a subject with a prostate cancer, said method comprising the step of administering to said subject a compound of item 19, or its pharmaceutical product, hydrate, pharmaceutically acceptable salt or any combination thereof, thereby treating a subject with a pancreatic cancer
31. A method of inhibiting a replication of a pancreatic cancer cell, said method comprising the step of administering to said subject a compound of item 19, or its pharmaceutical product, hydrate, pharmaceutically acceptable salt or any combination thereof, thereby inhibiting a replication of a pancreatic cancer cell.
32. A method of inhibiting a replication of a prostate cancer cell, said method comprising the step of administering to said subject a compound of item 19, or its pharmaceutical product, hydrate, pharmaceutically acceptable salt or any combination thereof, thereby inhibiting a replication of a pancreatic cancer cell.
33. A compound represented by the structure of formula XXXII: 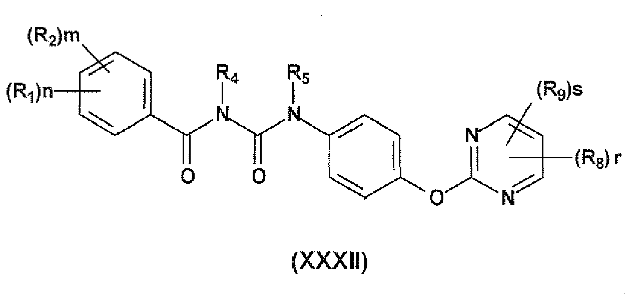 wherein, R₁ is NH₂, NHMe, NMe₂ or NO₂, and R₂ are independently H, NH₂, NO₂, CN,
   NHMe, NMe₂, CF₃, COOH, CONHOH, alkyl or aryl;
   R₄ and R₅ are independently, H ,alkyl, aryl or -SR₁₀, wherein R₁₀ is a substituted alkyl, cycloalkyl, aryl, heterocycle, or substituted phenyl fused with a saturated or unsaturated 4- to 6-membered rings optionally containing one to three heteroatoms independently selected from N,
   O, and S;
   R₈ and R₉ are independently H, alkyl or halogen;
   n is an integer between 1-5;
   m is an integer between 0-4;
   r is an integer between 1-3;
   s is an integer between 0-2;and
   wherein if R₁ is NO₂ in the ortho position, n is 1, m is 0, s is 0, r is 1, R₄ and R₅ are H, than R₈ is not iodine, Br or H in the para position.
34. The compound of item 31, wherein R₄ and R₅ are hydrogens
35. The compound of item 31, wherein R₈ is Br.
36. The compound of item 31, wherein R₁ is NH₂.
37. The compound of item 31, wherein R₁ is NO₂.
38. The compound of item 31, wherein R₈ is in the para position.
39. The compound of item 31, wherein R₁ is in the ortho position.
40. The compound of item 31, wherein s and m are 0.
41. A composition comprising the compound of item 31, or its isomer, pharmaceutically acceptable salt, *N*-oxide, hydrate or any combination thereof; and a suitable carrier or diluent.
42. A method of treating a subject with a pancreatic cancer, said method comprising the step of administering to said subject a compound of item 31, or its pharmaceutical product, hydrate, pharmaceutically acceptable salt or any combination thereof, thereby treating a subject with a pancreatic cancer.
43. A method of treating a subject with a prostate cancer, said method comprising the step of administering to said subject a compound of item 31, or its pharmaceutical product, hydrate, pharmaceutically acceptable salt or any combination thereof, thereby treating a subject with a pancreatic cancer.
44. A method of inhibiting a replication of a pancreatic cancer cell, said method comprising the step of administering to said subject a compound of item 31, or its pharmaceutical product, hydrate, pharmaceutically acceptable salt or any combination thereof, thereby inhibiting a replication of a pancreatic cancer cell.
45. A method of inhibiting a replication of a prostate cancer cell, said method comprising the step of administering to said subject a compound of item 31, or its pharmaceutical product, hydrate, pharmaceutically acceptable salt or any combination thereof, thereby inhibiting a replication of a pancreatic cancer cell.

### EXPERIMENTAL DETAILS SECTION

### MATERIALS

### Binding Studies

Tubulin assays with compounds represented by formula 6n: 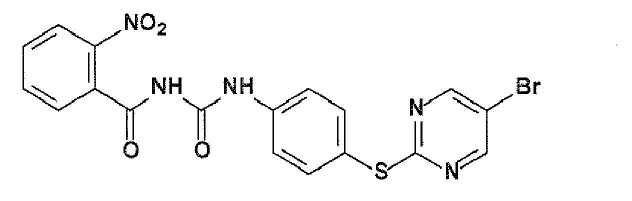 and formula 7d: 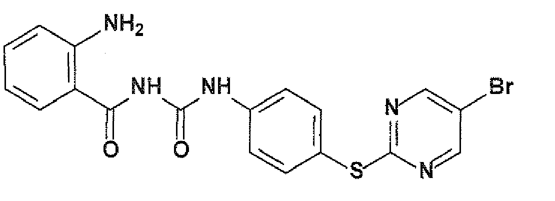 were performed. Briefly, tubulin polymerization was followed turbidimetrically at 350 nm in Beckman model DU7400 and DU7500 recording spectrophotometers equipped with electronic temperature controllers. Reaction mixtures contained 1.0 mg/mL tubulin (10 Amol/L), 0.8 mol/L monosodium glutamate, 4% DMSO, and varying drug concentrations. Samples were pre-incubated for 15 min at 30°C and chilled on ice. GTP (final concentration, 0.4 mmol/L) was added to each reaction mixture, and these were placed in cuvettes held at 0°C. Baselines were established, the temperature was raised to 30°C (∼0.5°C/s for 30 s), and polymerization was followed for 20 min. IC₅₀ values were determined by graphical interpolation of experimental points, with drug-containing samples compared with control reaction mixtures containing DMSO but no drug. At least three independent IC₅₀ values were obtained with each compound.

### Cell Lines and In vitro Culture Conditions

The cell lines ASPC1, MiaPaca2, HS766T, and L36PL were obtained from the American Tissue Culture Collection and are derived from pancreas carcinomas. The cell lines Panc1, Panc203, and Panc1005 are low-passage pancreas cell lines. The cell lines XPA3 and XPA4 are lowpassage. The cell lines E3LZ10 and E3JD13 are lowpassage pancreas cell lines. For clonogenic assessment, 500 cells per well were seeded in six-well plates with enriched RPMI (10% fetal bovine serum + 1% penicillin/streptomycin) and exposed to increasing drug concentrations for 96 h. After this, the drug was removed, and the cells were allowed to grow for 14 days before manual colony counting. Each experiment was done in duplicate for each drug concentration and was carried out independently at least thrice. For MAPT protein and mRNA expression analysis, the cell lines were grown in six-well plates with enriched RPMI. When the cells reached a 60% to 70% confluence, they were washed twice with chilled PBS; lysis buffer was added to the plates; and the cells were scraped for protein extraction. An identical experimental set was lysed with RLT (Qiagen) for mRNA extraction.

### In vivo Growth Inhibition Studies

Six-week-old female athymic nude mice (Harlan) were used. The research protocol was approved by the Johns Hopkins University Animal Care and Use Committee, and animals were maintained in accordance to guidelines of the American Association of Laboratory Animal Care. Surgical non-diagnostic specimens from patients who had undergone surgery at the Johns Hopkins Hospital were implanted s.c. in one to two mice for each patient, with two small pieces per mouse (F1 generation). Tumors were grown to 1.5 cm³ and then harvested, divided into small ∼3 x 3 x 3 mm pieces, and transplanted to another five mice, with two tumors per mouse (F2 generation). After a second growth passage, tumors were excised and propagated to cohorts of ≥20 mice. These groups constituted the F3, or treatment cohort. Tumors from this third mouse-to-mouse passage were allowed to grow to ∼200 mm3, at which time mice were randomized into three treatment groups, with five to six mice (10 evaluable tumors) in each group: control (vehicle), 6n (50 mg/kg i.p. twice per week), and 7d (50 mg/kg i.p. twice per week). Docetaxel treatment was given to selected groups at a dose of 30 mg/kg i.p. once a week for 2 weeks. Mice were monitored daily for signs oftoxicity and were weighed thrice per week. Tumor size was evaluated twice per week by caliper measurements using the following formula: tumor volume = [length x width²] / 2. Relative tumor growth inhibition was calculated by relative tumor growth of treated mice divided by relative tumor growth of control mice from the onset of therapy (T/C).

### Quantitative Real-time Reverse Transcription-PCR Analysis

Total RNA was extracted from cell pellets and tumors using the RNeasy Mini kit (Qiagen). cDNA was synthesized using the iScript cDNA synthesis kit (Bio-Rad), following the manufacturer's instructions. Relative quantification ubiquitin C mRNA was achieved using an iCycler iQ realtime PCR detection system (Bio-Rad) using ABI Taqman probes. Accumulation of the specific PCR products was detected as an increase in fluorescence that was plotted against cycle number to determine the CT values. Relative expression of the mRNA analyzed was estimated using the formula: 2^{ΔCT}, where DC_{T} = CT (mRNA) - C_{T} (ubiquitin C). Samples were analyzed in a blinded manner.

### Western Blot Analysis

Equal amounts of protein were resolved on 10% polyacrylamide gels. Gels were transferred onto nitrocellulose membranes that were incubated overnight at 4°C with antibodies against MAPT and actin (Santa Cruz Biotechnology). The immunoreactive proteins were detected using the enhanced chemiluminescence method (Amersham).

### DNA Sequencing Analysis

DNA was extracted from the cell line pellets and frozen tumors, tested by means of selection with specific primers, amplification with PCR, and sequencing of the different exons and introns that constitute the gene and are known to harbor mutations (MAPT: exons 9-13) or the complete gene (TUBB: exons 1-4). The following primers were designed/used to sequence the MAPT gene: exon 9, forward 5'-CTGCCTAACCCAGTGGTGA-3' and reverse 5'-CACAGTCCCACGACTCCAC-3'; exon 10. forward 5'-CTCTGCCAAGTCCGAAAGTG-3' and reverse 5'- TCCTGAGAGCCCAAGAAGG-3'; exon 11, forward 5'-ATAAGGGCCTGGGCTTACAC-3' and reverse 5'-CCGCAAGTTTCACACTCAAC-3'; exon 12, forward 5'-TTCAAGTCCTGCTGCAAACC-3' and reverse 3'-CTCCAGGTGATTGAGACAGG-3'; exon 13, forward 5'-AGGCTGCAGTGAGGTGAGAT-3' and reverse 5'-CCGAGTGACAAAAGCAGGTT-3' (Invitrogen/Life Technologies). The following primers were designed/used to sequence the TUBB gene: exon 1, forward 5'-TACATGACCGGCATCGACTA-3' and reverse 5'-ACAAACTTGAGAGGGGCAAA-3'; exons 2 and 3, forward 5'-AGCGAGACTCCGTCTCAAAA-3' and reverse 5'-CTTTTGCTGTGTCCTTGCAC-3'; exon 4a, forward 5'-CGAGGGAATTATTTGAAAAGTTG-3' and reverse 5'-GGCATCGAAGACCTGCTG-3'; exon 4b, forward 5'-TTCTTTATGCCTGGCTTTGC-3' and reverse 3'-CCCAGAATGGCAGAAACCTA-3'; exon 4c, forward 5'-TCCCTCAGAATTTGTGTTTGC-3' and reverse 5'-GCTCGAGTGAGGGAGGTAGA-3V; exon 4d, forward 5'-TGTCAGCAGTATTATCTCCACTTT-3' and reverse 5'-TTAAGTGTCAATTTAAGGAGA-3' (Invitrogen/Life Technologies). The 25-µL PCR reaction consisted of 0.2 µmol/L concentration of each outside primer along with 150 ng of genomic DNA, HotMaster Mix 2.5x (Eppendorf) containing 960 µmol/L deoxynucleotide triphosphates, 54 mmol/L KCl, 3.0 mmol/L MgCl2, and 0.6 units of Taq Polymerase. Thermocycler conditions were initial cycle at 95°C for 3 min, followed by 10 cycles 94jC for 45 s, 57.6jC for 1 min, and 72°C for 1 min; 20 cycles of 89°C for I min, 57.6°C for 1 min, and 72°C for 1 min. PCR product clean-up consisted of 3 µL Shrimp Alkaline Phosphatase and 0.3 AL Exonuclease1 (GE Healthcare) per sample incubated at 37°C for 1 h followed by one step of 80°C for 15 min. The existence of a variant was determined by direct nucleotide sequencing. Sequencing was done using an ABI 3730XL Sequencer (Applied Biosystems), following standard dideoxy terminator protocols. Sequences were analyzed using Sequencher (Genecodes). Only changes present in both strands were identified as single nucleotide polymorphisms.

### Statistical Analysis

To assess the treatment effect, a hierarchical linear regression model was used with random intercepts and fixed slopes. Based on the results of exploratory plots, the assumption of fixed effects for slopes seemed reasonable. This model allowed for mouse effects to be nested within patient effects so that the hierarchical structure is fully accounted for. Specifically, the following linear regression model was fitted: log (*y_{iJt}*) = a_{1*ij*}time*ᵢⱼₜ* + b_{2*j*}time*ᵢⱼₜ* x trt*ᵢⱼ* + βtime²*ᵢⱼₜ* + e*ᵢⱼₜ* where *j* indexes patient, *i* indexes mouse, and t indexes time; y*ᵢⱼₜ* is the percentage change in tumor volume (compared with day 0) in mouse *i*, which is a xenograft for patient *j* at time *t*; and trt*ᵢⱼ* is treatment (1 = treated, 0 =control) received by mouse *i*, which is a xenograft for patient *j*. A series of models to determine a good fitting model were explored and found that the above model was adequate. An intercept and main effect of treatment were not included in the model that makes two assumptions: (a) at time 0, the change from baseline is 0 (which is empirically true), and (b) at baseline, the treated and untreated curves intersect. The model was fit using Win-Bugs software that estimates models using a Markov chain Monte Carlo estimation procedure. A burn-in of 10,000 iterations was done, and then the chain was run for an additional 20,000 iterations, of which every 10^{th} iteration was saved for inference. Several chains were run with different starting values to ensure convergence. No convergence issues arose. The fitted model was used to make inferences with respect to the treatment effect and to create fitted regression lines (rescaled on the Y-axis for clearer interpretation). Point estimates of variables of interest (e.g., expected difference in tumor volume between treated and control at 28 days) and 95% credible intervals were based on the mean of posterior distributions and the 2.5th and 97.5th quantiles of the posterior distribution, respectively. The comparisons between means and proportions obtained from the biological studies were done using Student's *t*-test and χ² method, respectively. ANOVA was used when three or more variables were compared.

### EXAMPLE 1: GENERAL METHOD FOR SYNTHESIS OF BPU SULFUR ANALOGS

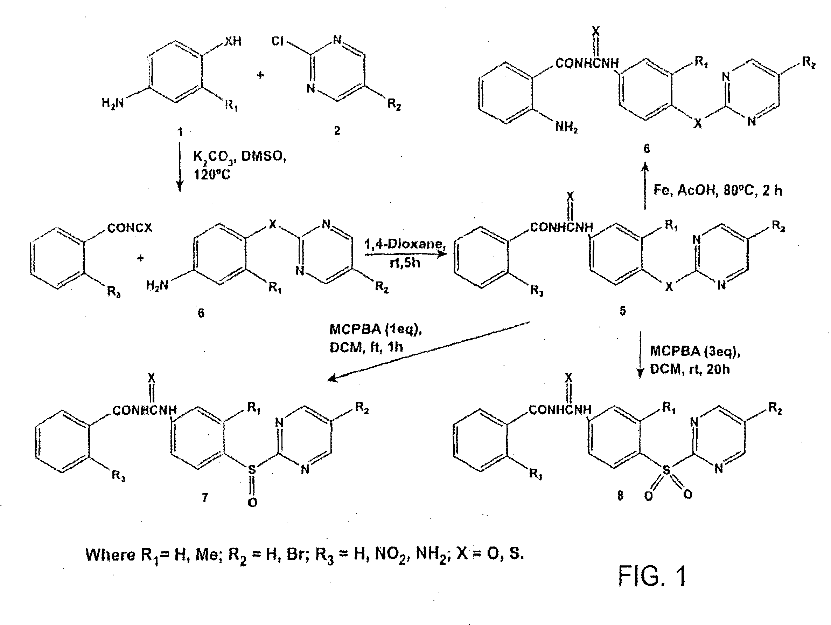

These new analogs were synthesized in excellent yield by coupling corresponding benzoylisocyanate or benzoylisothiocyanate and aniline derivatives. Sulfur analogs of aniline intermediate were synthesized by reaction of substituted amino thiophenol with aryl halide. Sulfoxide and sulfone derivatives were prepared by oxidation of sulfides (Figure 1). All the compounds were characterized by NMR and LC-MS studies.

Unless otherwise noted, reactions were run in flame-dried round-bottomed flasks under an atmosphere of uitra-high-purity (UHP) argon. All reactive liquid reagents were transferred by syringe or cannula and were added into the flask through a rubber septum. Tetrahydrofuran was freshly distilled from sodium benzophenone ketyl immediately prior to use. All other solvents and reagents were used as received unless otherwise stated. n-BuLi was obtained from commercial sources and was titrated with N-pivaloyl-O-toluidine prior to use. Diethyl ether (ether) and tetrahydrofuran (THF) were distilled from sodium benzophenone ketyl prior to use. Methylene chloride (CH₂C₁₂) was distilled from calcium hydride prior to use. All other compounds were purchased from Aldrich Chemical Co. and used without further purification. Analytical thin-layer chromatography (TLC) was conducted with silica gel 60 F254 plates (250 lm thickness; Merck). Column chromatography was performed using short path silica gel (particle size <230 mesh), flash silica gel (particle size 400-230 mesh), or Florisil (200 mesh). Yields are not optimized. Purity of products was judged to be >95% based on their chromatographic homogeneity. High-performance liquid chromatography (HPLC) was carried out with a Rainin HPLX system equipped with two 25 mL/min preparative pump heads using Rainin Dynamax 10 250 mm (semipreparative) columns packed with 60 A ° silica gel (81m pore size), either as bare silica or as C-18-bonded silica. Melting points were measured using a Mel-Temp metal-block apparatus and were uncorrected. Nuclear magnetic resonance (NMR) spectra were obtained either on a Varian XL-400 spectrometer, operating at 400 MHz for 1 H and 100 MHz for 13C, or on a Varian XL-500 spectrometer, operating at 500 MHz for 1H and 125 MHz for 13C. Chemical shifts are reported in parts per million (ppm, δ) downfield from tetramethylsilane (TMS). Multiplicities of signals in the 1H NMR spectra are reported as follows: s (singlet), d (doublet), t (triplet), q (quartet), m (multiplet), dd (doublet of doublets), dt (doublet of triplet), etc.

Infrared spectra were obtained on a Perkin Elmer 1600 FT-IR spectrometer as liquid films and thin layer with NaCl cells. Intensities were reported as s (strong 67-100%), m (medium 34-66%), and w (weak 0-33%) with the following notations, br (broadened), sh (shoulder), etc.

Optical rotations were recorded on JASCO, P-1100 model polarimeter (Japan Spectroscopic Co., Ltd.) with sodium D line at the temperatures as indicated in the experimental section for the specific compounds.

Analytical Thin Layer Chromatography (TLC) was performed on Merck silica gel plates (Merck Kieselgel, 60, 0.25-mm thickness) with F254 indicator. Compounds were visualized under UV lamp and/or by developing with iodine, vanillin, p-anisaldehyde or KMnO4 followed by heating with a heat gun. Flash chromatography (Still, W. C., et al., J. Org. Chem., 43:1404 (1978)) was performed as reported by Still and coworkers on 230-400 mesh silica gel (E.M. Science) with technical and/or HPLC grade solvents. Medium Pressure Liquid Chromatography (MPLC) was performed with FMI pump and prepacked silica gel column (Merck, Labor Columns, LiChroprep Si 60, 40-63 □m). High Pressure Liquid Chromatography (HPLC) was performed on a Rainin HPLX system equipped with two 25 mL pump heads and a Rainin Dynamax UV-C dual-beam variable wavelength detector set at 254 or 260 nm using Phenomenex, Luna 5 µ C18 semipreparative (250 x 10 mm) column and Chiralcel OJ semipreparative (250 x 10 mm) column.

Low and high-resolution mass spectra (LRMS and HRMS) were obtained with electronic or chemical ionization (EI or CI) either (1) on a VG Instruments 70-S spectrometer run at 70 eV for EI and run with ammonia (NH₃) as a carrier gas for CI or (2) on a Finnigan- MAT CH5, a Finnigan-MAT 731, or a VG Instruments 70-VSE spectrometer run at 70 eV for EI and run with methane (CH4) for CI.

### Chemistry

Novel sulfur derivatives of BPU were synthesized as shown in Figure 1. Aniline derivatives 4 were prepared by reaction of substituted aminophenols or amino thiophenols with substituted 2-chloropyrimidines in the presence of K₂CO₃ and DMSO.8 Condensation of substituted benzoyl isocyanates or benzoyl isothiocyanates with aniline derivatives 4 gave a series of BPU and benzoylphenylthiourea (BPTU) analogues 6a-n. Reduction of the 2-NO2 group was performed using Fe and AcOH 14 to obtain the 2-NH2 derivatives 7a-d. Sulfoxide and sufane derivatives 8a-h were prepared from the corresponding sulfide by oxidation with MCPBA (Heynderickx, A., et al.,. Synthesis, 1112-1116 (2003)) followed by chromatographic purification. All the compounds were characterized by spectral data analysis that confirmed the assigned structures.

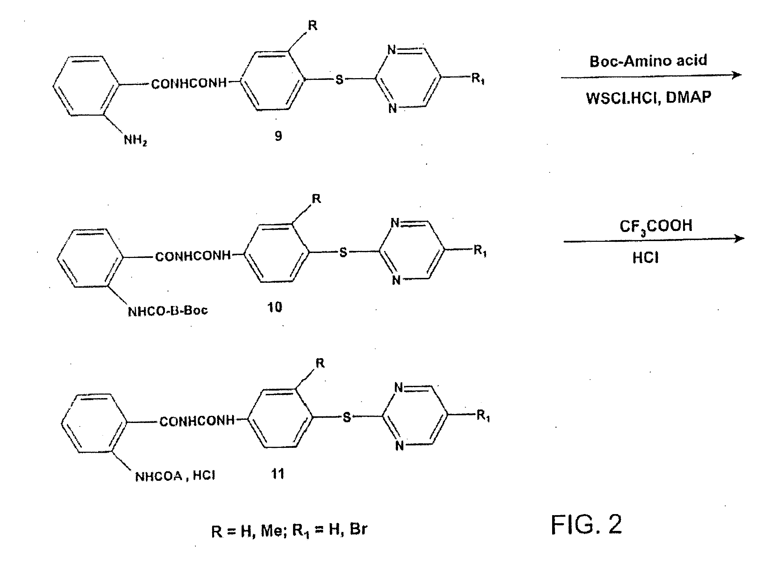

Ring-A aniline modified amino acid prodrugs were prepared according to figure 2. The synthetic strategy involves coupling of Boc-protected amino acid with ring-A amine on the BPU compounds using coupling reagent 1-ethyl-3-(3-dimethylaminopropyl)-carbodiimide hydrochloride (WSCI.HCl). Finally Boc protection was removed by treating with TFA.

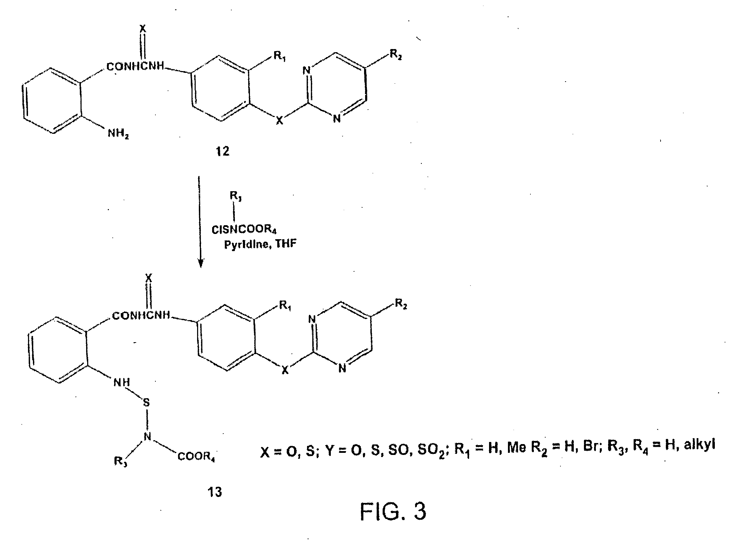

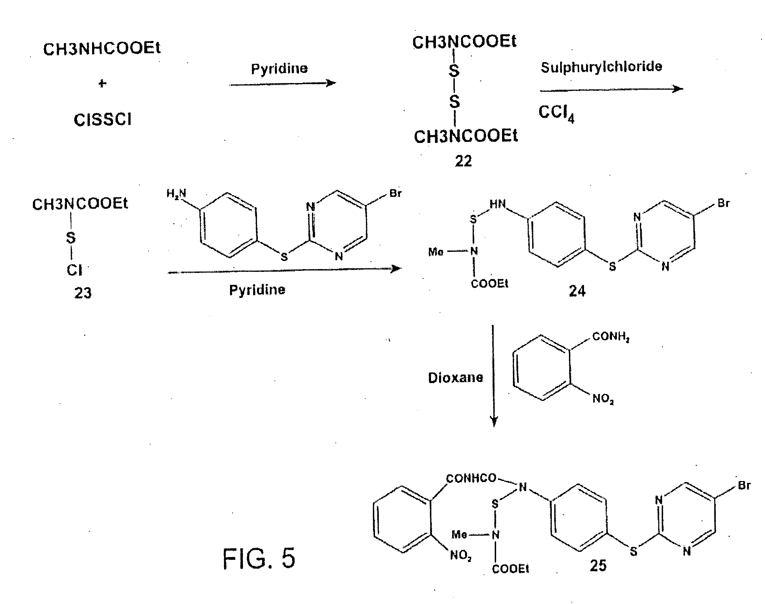

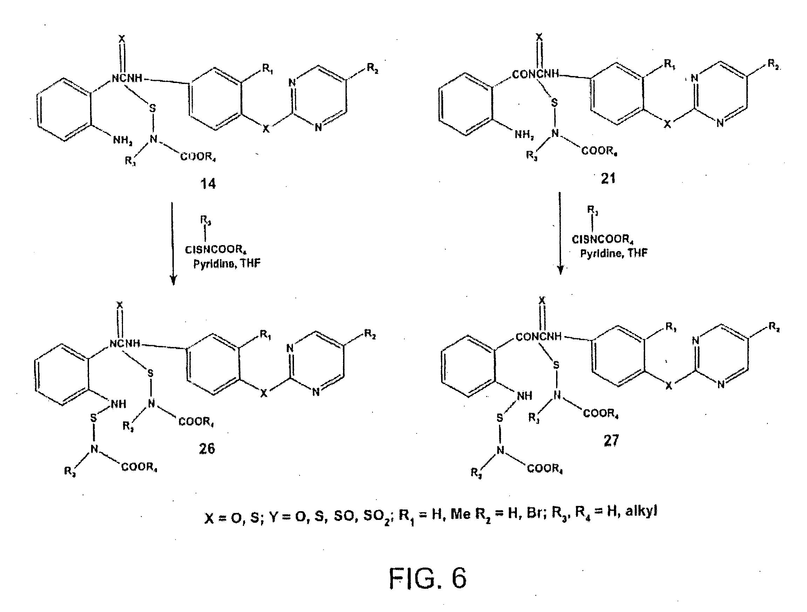

Ring-A aniline modifed carbamylosulfenyl derivative of BPU sulfur analog was prepared according to figure 3. To prepare urea and benzoyl urea modified prodrugs, scheme 4 was used. Compound 15 was prepared by treating 14 with oxalyl chloride and then coupled with 17 and 19 in dioxane to obtain prodrugs of general structure 20 and 21 respectively. Representative example has been shown in figure 5. Both ring-A and urea modified prodrugs can be prepared according to figure 6.

BPU compounds have very low solubility in water and limited solubility in common organic solvents. Therefore, the modification of these compounds to improve their bioavailability is expected to lead to compounds with better biological properties.

The lead prodrugs from the in vitro and in vivo studies are subjected to pharmacokinetic analysis to study stability, clearance and metabolism profiles.

### Synthesis of Compounds 6a-n, 1-[4-(5-Bromopyrimidin-2-ylsulfanyl)phenyl]-3-(2-nitrobenzoyl)-urea (6n)

A solution of 5-bromo-2-chloropyrimidine (5 g, 0.026 mol), 4-aminothiophenol (3.24 g, 0.026 mol), and K₂CO₃ (7.14 g, 0.052 mol) in dry DMSO (50 mL) was stirred at 120 °C for 2.5 hours under N2. After cooling, the reaction mixture was poured into water and extracted with ethyl acetate. The organic layer was washed with water and saturated brine and then dried. The solvent was evaporated to give a residue that was purified by silica gel flash column chromatography (ethyl acetate-hexane 1:3) to give 4-(5-bromopyrimidin-2-ylsulfanyl)phenylamine (4n), yield 74%. 1H NMR (400 MHz, CDCl3): δ 8.51 (s, 2H), 7.37 (d, J) 8.0 Hz, 2H), 6.72 (d, J) 8.0 Hz, 2H), 2.63 (s, 2H); EI-MS m/z 281 [M]+, 283 [M + 2]+.

A solution of 2-nitrobenzoyl isocyanate (3 g. 0.016 mol) in dry 1,4-dioxane (15 mL) was added dropwise to a solution of 4n (2.93 g, 0.01 mol) in dry 1,4-dioxane (15 mL) with stirring at room temperature. The reaction mixture was stirred for 18 hours and then diluted with water. The precipitated solid was collected by filtration and washed with water. The solid was dissolved in ethyl acetate, and the organic layer was washed with water 2-3 times, dried, and concentrated to give 1-[4-(5-bromopyrimidin-2-ylsulfanyl)- phenyl]-3-(2-nitrobenzoyl)urea (6n), yield 92%. 1H NMR (400 MHz, DMSO-d6): δ 11.32 (br, s, 1H), 10.35 (br, s, 1H), 8.78 (s, 2H), 8.22 (m, 1H), 7.91 (m, 1H), 7.77 (m, 2H), 7.67 (m, 2H), 7.57 (m 2H); EI-MS m/z 473 [M]+, 475 [M+2]+; HRMS calculated for C18H12BrN5O4SNa [M+Na]+: 495.9685, found: 495.9701. Anal. (C18H12BrN504S) C, H, N.

### Synthesis of Compounds 7a-d

Iron powder (1.77 g, 31.63 mmol) was added in portions to a mixture of 6n (3 g, 6.32 mmol) in AcOH (90 mL) at 80 °C. The reaction mixture was refluxed for 30 min and then cooled to room temperature and diluted with water. The precipitated solid was collected by filtration. The solid was dissolved in an excess of ethyl acetate and filtered. The filtrate was dried and concentrated to give a residue that was purified by silica gel flash column chromatography (ethyl acetate-hexane 2:3) to give 7d, yield 62%. 1H NMR (400 MHz, DMSO-d6): δ 11.92 (br, s, 1H), 10.73 (br, s, 1H), 8.75 (s, 2H), 7.68 (m, 2H), 7.57 (m, 2H), 7.25 (m, 1H), 6.79 (m, 1H), 6.59 (m 2H); EI-MS m/z 443 [M]+, 445 [M + 2]+; HRMS calculated for C18H14BrN5O2SNa [M + Na]+: 465.9943, found: 465.9938. Anal. (C18H14BrN5O2S) C, H, N.

### Synthesis of Compounds 8a-h

To a stirred solution of 6n (1.0 g, 2.11 mmol) in DCM (50 mL) at 0 °C was added MCPBA (0.364 g, 2.11 mmol) in portions. The resulting mixture was stirred at room temperature for 6 h and then diluted with water and made slightly basic with Na₂CO₃ solution. The DCM layer was separated, and the aqueous layer was extracted with DCM. The combined organic layers were dried and evaporated to give a residue that was purified by silica gel flash column chromatography (ethyl acetate-methanol 5:1) to give 8g, yield 48%. 1H NMR (400 MHz, DMSO-d6): δ 11.32 (br, s, 1H), 10.38 (br, s, 1H), 9.15 (s, 2H), 8.20 (m, 1H), 7.88 (m, 1H), 7.72-7.80 (m, 6H); EI-MS m/z 489 [M]+, 491 [M + 2]+; HRMS calculated for C18H12BrN5O5SNa [M + Na]+; 511.9634, found: 511.9632. Anal. (C18H12BrN5O5S) C, H, N.

### 1-[4-(5-Bromopyrimidine-2-sulfonyl)phenyl]-3-(2-nitrobenzoyl)-urea (8h)

The title compound was synthesized from 6n according to above procedure using 3 equiv of MCPBA, yield 56%. 1H NMR (400 MHz, DMSO-d6): δ 11.42 (br, s, 1H), 10.55 (br, s, 1H), 9.23 (s, 2H), 8.21 (m, 1H), 7.76-7.97 (m, 7H); EI-MS m/z 505 [M]+, 507 [M + 2]+; HRMS calculated for C18H12BrN5O6SNa [M +Na]+: 527.9583, found: 527.9592. Anal. (C₁₈H₁₂BrN₅O₆S) C, H. N.

The terms and expressions which have been employed herein are used as terms of description and not of limitation, and there is no intention in the use of such terms and expressions of excluding equivalents of the features shown and described, or portions thereof, it being recognized that various modifications are possible within the scope of the invention claimed. Moreover, any one or more features of any embodiment of the invention may be combined with any one or more other features of any other embodiment of the invention, without departing from the scope of the invention. For example, the features of the compounds of this invention are equally applicable to the methods of treating disease states described herein. All publications, patents, and patent applications cited herein are hereby incorporated by reference in their entirety for all purposes.

### EXAMPLE 2: CYTOTOXIC EVALUATION OF BPU SULFUR ANALOGS

Eight of these analogs 6d, 6h, 6n, 6g, 7b, 7d, 8g, and 8h were tested for their cytotoxicity in seven pancreatic cell lines ASPC1, Pancl, Panc203, Panc430, Pane 1005, MiaPaca2, and HS766T using MTT assay. Cells were trypsinized, seeded at 5x10³ cells/well in 96-well plate and allowed to grow for 24 hours before the treatment with exponential increasing concentrations of drugs in the presence of 10% FBS. After a 96-hours period of treatment, 20 µl of MTT solution (5 mg/ml in PBS) were added to each well, and the plates were then incubated for 3 hours at 37°C. Medium was then replaced with 100 µl of DMSO per well. Plates were shaken and the optical density was measured at 570 nm using a multiwell plate reader (Bio-Rad, Model 550, Bio-Rad Inc., Hercules, CA). Each experiment was performed in triplicate for each drug concentration and was carried out independently at least 3 times. The IC₅₀ value was defined as the concentration needed for a 50% reduction in the absorbance calculated based on the survival curves. Response to drug treatment was assessed by standardizing treatment groups to untreated controls. Compounds activity was compared with compound 1. Two compounds 6n and 7d found to be 20 time more potent than compound 1.

### Growth Inhibition Results

Growth inhibition of pancreatic cell lines by compound 1 and the compounds 6n, 7d, 6d, 6h, 7b, 6g, 8g, 8h of the present invention are depicted in following figures 8-16, respectively.

### Growth Inhibition Assay for Prostate Cancer Cell lines

This MTT assay was carried using human prostate cancer cell lines CWR22R, LAPC-4 and LnCap using Promega cell titer 96 nonradioactive cell proliferation kits. Cells were trypsinized and centrifuged at 1000 rpm for 5 minutes and the supernatant was aspirated. Cells were resuspended with HBSS to a concentration of 10⁶/ml. Cell suspension was diluted in serum containing medium to plate 2000 cells/well/0.21ml. Cell suspension was transferred to sterile solution basin. Cells were plated using multichannel pipette set at 200 µl and incubated overnight for attachment. One plate was prepared for day 0 MTT assay as follows: 100 µl of media was removed from each well using multichannel pipette and 15 µl of MTT reagent was added. Plates were incubated at 37°C for 4 hrs. 100 µl of STOP solution was added to each well and incubated at room temperature for 1 hour. Absorbance was measured at 570 and 650 nm on plate reader. All the media from the remaining plates were carefully removed and the cells were treated with the media containing drug. MTT assays were done on days 0, 3, 5 and 7. In the preliminary screening four BPU sulfur analogs 61, 6h, 6n, 7d and 8h were screened and their activity was compared with compound 1. Two compounds 6n and 7d found to be 20 times more potent compare to compound 1 compound. The data is shown in the following figures17-34.

### In-vitro growth inhibition analysis of compounds 6n and 7d and correlation between putative markers and efficacy

Both compounds 6n and 7ds showed similar effects on the 11 pancreatic cancer cell line panel. Clonogenic growth was impaired at concentrations between 0.01 and 0.5 µmol/L (Fig. 35B), with no colony formation at higher doses in 9 of the 11 cell lines. MAPT mRNA levels varied over a wide (65,000-fold) range (Fig. 35C). Expression was normalized to the lowest sample, which was arbitrarily assigned a value of 1. There was a correlation between mRNA and protein, with the three lowest mRNA-expressing cells lines showing a low protein expression. Two isoforms of MAPT protein were detected (45 and 65 kDa). The three cell lines with lower MAPT mRNA levels expressed traces of the 65-kDa isoform. The 45-kDa isoform predominates in E3LZ10, ASPC1, Panc203, and Panc1005 (the second highest MAPT-expressing cell line), whereas the 65-kDa isoform predominates in XPA4, E3JD13, and MiaPaCa2 (the highest MAPT-expressing cell line). The two MAPT isoforms seemed equally expressed in L36PL.

At the most discriminative concentrations of both compounds (0.01 and 0.1 µmol/L), the three most sensitive cell lines were consistently those with lower MAPT levels (Panc 1. HS766T, and XPA3) and showed a 10-fold superior sensitivity than those with higher levels of MAPT. In the eight high protein-expressing cell lines, no correlation between isoform expression and activity was detected. In Fig. 35D, the clonogenic growth at 0.1 µmol/L for both compounds and the MAPT mRNA levels of the cell lines is depicted. Treatment did not induce variations in MAPT mRNA levels.

### Biology

Table 2 (Growth Inhibition of Pancreatic Cancer Cell Lines by BPU and BPTU Analogues (IC50)a)compares the cytotoxicity of a series of new derivatives against a panel of seven pancreatic cancer cell lines, as determined by MTT assay. See, Mosmann, T., J. Immunol. Methods 65:55-63 (1983).

**Table 2**

| compd | ASPC1 | Panc1 | Panc203 | Panc430 | Panc1005 | Miapaca2 | HS766 |
|---|---|---|---|---|---|---|---|
| **1** | 0.85 | 0.70 | 0.95 | 0.80 | 5.00 | 0.85 | 1.00 |
| **6g** | >10 | >10 | 7.50 | 2.00 | >10 | 5.00 | 5.50 |
| **6h** | 10.00 | 9.50 | >10 | 7.50 | >10 | 10.00 | 8.50 |
| **6m** | 0.77 | 0.68 | >10 | 10.00 | >10 | 0.74 | 0.47 |
| **6n** | 1.00 | 0.085 | 0.70 | 0.09 | 10.00 | 0.086 | 0.088 |
| **7b** | 6.0 | 7.5 | 5.5 | 6.5 | >10 | 7.0 | 8.0 |
| **7c** | 5.20 | 3.50 | >10 | >10 | >10 | 0.92 | 0.27 |
| **7d** | 0.45 | 0.09 | 0.085 | 0.20 | 10.00 | 0.095 | 0.70 |
| **8g** | 2.50 | 6.50 | 5.50 | 5.50 | 5.50 | 8.00 | 6.50 |
| **8h** | >10 | >10 | >10 | >10 | >10 | 6.10 | >10 |

| | | | | | | | |
|---|---|---|---|---|---|---|---|
| ^{a}IC₅₀ values expressed in µM; Average of three independent experiments. | | | | | | | |

Of all the tested compounds, compound 6n possessed the highest potency, with IC₅₀ values 0.085, 0.09, 0.086, and 0.088 µM against the Panel, Pauc430, Miapaca2, and HS766 cell lines, respectively. Results with compound 7d demonstrated that substitution of NH2 for the NO2 group (6n) in the benzoyl ring did not diminish the cytotoxic activity against the Panc1, Panc203, and Miapaca2 cell lines. Compounds 6n and 7d were 7- to 11-fold more potent than 1 against these cell lines. In the presence of 6m, the growth of cell lines ASPC 1. Panc1, Miapaca2, and HS766 was inhibited, with IC₅₀ values of 0.77, 0.68, 0.74, and 0.47 µM, respectively.

Cell lines Miapaca2 and HS766 were susceptible to 7c, with IC50 values of 0.92 and 0.27 µM, respectively. Compound 8g was effective in inhibiting the growth of all the cell lines, with IC₅₀ values ranging from 2.5 to 8.0 µM. For the remaining compounds, the IC₅₀ was >10 µM for all the cell lines. Compounds with significant inhibitory activity in pancreatic cancer cells were further evaluated against prostate cancer cell lines in the MTT assay, (Mosmann, T., J. Immunol. Methods 65:55-63 (1983)) and the results were compared to those for compound 1 (Table 3). Table 3 demonstrates the growth inhibition of prostate cancer cell lines by BPU and BPTU Analogues (IC50)^{a}.

**Table 3**

| compd | CWR22R | LnCaP | LAPC-4 |
|---|---|---|---|
| **1** | 1.00 | 0.50 | 0.25 |
| **6h** | >10 | >10 | 6.00 |
| **6l** | 5.00 | 0.50 | 0.60 |
| **6m** | 5.00 | 1.00 | 0.20 |
| **6n** | 0.05 | 0.05 | 0.05 |
| **7c** | 2.00 | 0.75 | 0.75 |
| **7d** | 0.50 | 0.50 | 0.10 |
| **8h** | >10 | 0.50 | 4.00 |

| | | | |
|---|---|---|---|
| ^{a}IC₅₀ values expressed in µM; Average of three independent experiments. See, Mosmann, T., J. Immunol. Methods 65:55-63 (1983). | | | |

Compound 6n was the most highly potent of the tested compounds, with an TC₅₀ of 0.05 µM against all prostate cell lines. It was 5- to 20-fold more potent than compound 1. The CWR22 and LnCap cell lines were equally sensitive (IC₅₀ ) 0.5 µM) to the growth inhibitory effects of 7d. 7c also inhibited cell growth in the prostate cancer cell lines LnCap and LAPC-4 (IC50) 0.75 µM).

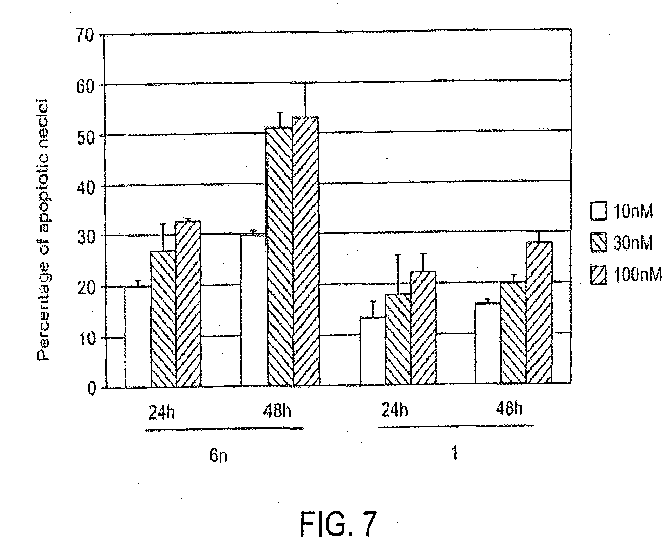

Compound 6n treatment of MCF-7 cells for 48 hours produced rates of apoptosis of 29.7% (10 nM), 51.2% (30nM), and 53.6% (100 nM), respectively, as compared to 16.1%, 19.5%, and 27.7% for 1 at the same concentration. Apoptosis of MCF-7 cells by 6n and 1 are demonstrated in figure 7 and 6n was more potent than 1 in killing MCF-7 cells (Zhu, T., et al., Cancer Res, 65:317-324 (2005)).

A mechanism-based tubulin assembly analysis (Hamel, E., Cell Biochem. Biophys., 38:1-22 (2003); and Verdier-Pinard, P., et al., Mol. Pharmacol, 53:62-76 (1998)) of four compounds from this series was also conducted. As shown in Table 4, 6n and 6m were effective in inhibiting tubulin assembly, with IC₅₀ values of 2.1 and 4.7 µM, respectively. These values were comparable to the IC₅₀ of 1.7 µM for the drugs combretastatin and podophyllotoxin, and 2.7 µM for colchicine, which have a similar mechanism of action. Present data indicates (Table 4) that none of our compounds bound to the colchicine binding site on the microtubules.

**Table 4**

| Inhibition of Tubulin Polymerization and Colchicine Binding | | |
|---|---|---|
| compd | tubulin^{a} IC₅₀ ± SD (µM)± | colchicine binding^{b} (% ± SD) |
| **6m** | 4.7 ± 0.3 | 21 ± 5 |
| **6n** | 2.1 ± 0.5 | 17 ± 3 |
| **7c** | 36 ± 0.7 | nd *c* |
| **7d** | >40 | nd |
| combretastatin A4 | 1.7 ± 0.2 | 94 ± 5 |
| podophyllotoxin | 1.7 ± 0.1 | 77 ± 8 |
| colchicines | 2.7 ± 0.4 | |

| | | |
|---|---|---|
| ^{a} Inhibition of tubulin polymerization. Tubulin was at 10 µM (Hamel, E., Cell Biochem. Biophys., 38:1-22 (2003)). ^{b}Inhibition of [³H]colchicine binding, Tubulin was at 1 µM; both [³H]colchicincines and inhibitor were at 5 µM (Verdier-Pinard, P., et al., Mol. Pharmacol, 53:62-76 (1998)). ^{c} No data. | | |

### In vivo Dose-Finding Study of compounds 6n and 7d

Both compounds were explored by the i.p. route in cohorts of six mice each, with a duration of treatment of 4 weeks. The two initial dose levels of both compounds (20 mg/kg twice per week and 30 mg/kg twice per week) were not associated with noticeable toxicity. Therefore, 50 mg/kg twice per week was explored. One of six mice in the 6n group had a weight loss of 15% by the 4th week, but no deaths occurred. Therefore, the dose was escalated to 54 mg/kg thrice per week. In the 6n and 7d groups, respectively, two and one of the mice died in the 3rd week, and three of four and four of five of the remaining animals loss >15% of their weight. Thus, for 28-day efficacy studies following an i.p. route, we selected 50 mg/kg twice a week for both compounds.

### In vivo Growth Inhibition Analysis

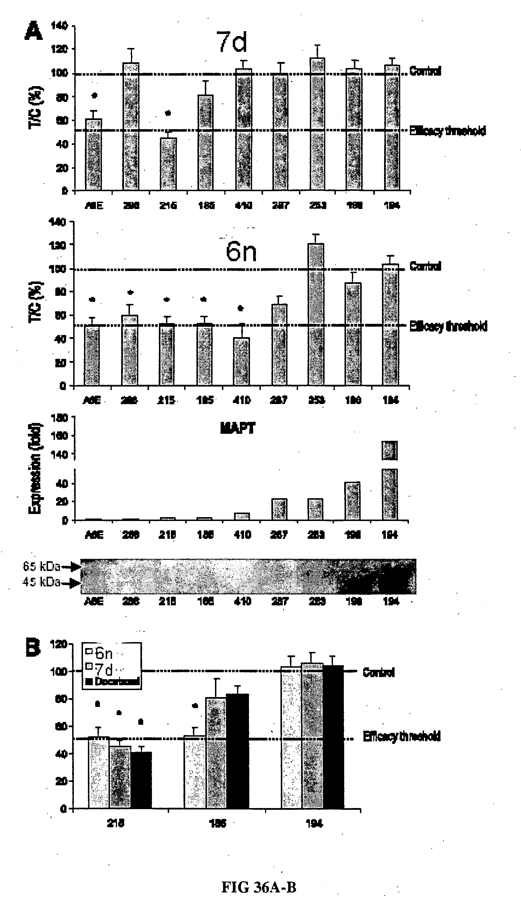

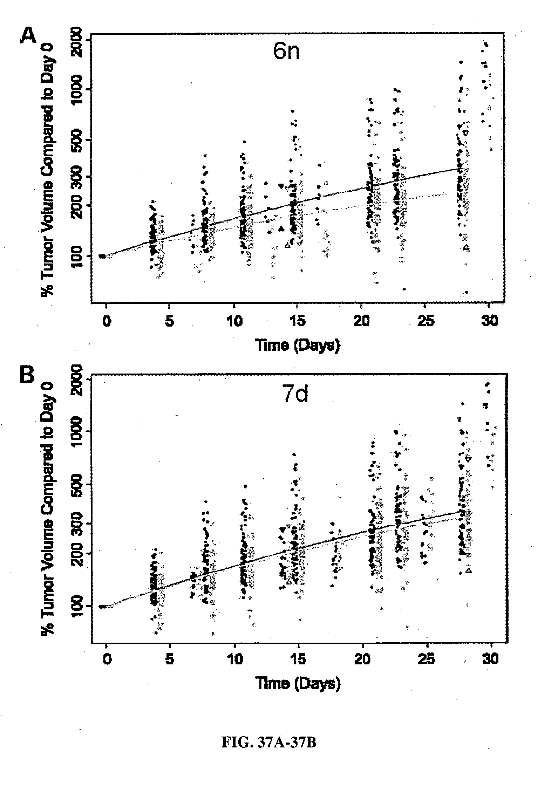

Tumors from nine cases of pancreatic cancer were used to establish xenografts in nude mice, and the animals were treated with either vehicle, 6n, or 7d (Fig. 36A). The level of efficacy varied both between drugs and within cases treated with each drug. Figure 37 provides an integrated summary of all data points obtained in the studies. Overall, there were no statistical differences between the slopes of the treated versus control group for either of the agents, although 6n showed a greater efficacy than 7d. Assessment of individual groups, as shown in Fig. 36A, shows that in 6n-treated tumors, four cases had an average growth after 28 days of treatment of ≤50%, and a fifth had 60%. In contrast, 7d induced significant growth arrest in only two cases with growths of 43% and 59%.

### In vivo Correlation between MAPT and Efficacy

To gain insight into factors that could at least partially explain the observed observations, we determined the expression of MAPT in the patient tumor xenografts. Expression was normalized to the lowest sample, which was arbitrarily assigned a value of 1. There was a correlation between mRNA and protein, with the three highest mRNA-expressing cases showing significant protein expression by Western blot. The five cases with the lowest MAPT expression were those where 6n showed a tumor growth reduction that was statistically significant compared with their respective control groups; or, inversely, in all cases with low MAPT levels, 6n was able to significantly inhibit tumor growth. However, one of those, despite being significant, was not considered relevant, as the T/C was 60%. The comparison of the proportion of sensitive cases according to having below (4 of 5) or above (0 of 4) the median MAPT expression is statistically significant (two-sided Fisher's test, P = 0.048). 7d induced growth arrest in two cases with minimal expression of MAPT, but this trend is weaker than with 6n, as fewer cases were sensitive. Consequently, the predictive value for 7d is lower.

### Comparison of 6n and 7d In vivo Activity with Docetaxel

Three of the cases were also treated with docetaxel to compare the efficacy of the benzoylphenylurea agents with an established anticancer agent targeting microtubules and also to determine the correlation of such efficacy and MAPT expression (Fig. 36B). Docetaxel, as was the case with both 6n and 7d, was particularly efficacious in 215, a low MAPT-expressing case. Docetaxel showed lower efficacy than 6n in 185, and all three compounds were inactive in 194, a case with the greatest expression of MAPT among those examined.

### Analysis of Class III β-Tubulin Expression and MAPT and Class I β-Tubulin Mutations

The level of expression of class III β -tubulin was also assessed in the cell lines and in the tumors. Although among the lowest expressing cell lines for class III β -tubulin were HS766T and Panc1, and the highest was MiaPaCa2, the correlation for the group of cell lines as a whole was poor (Fig. 35E), and there was not a correlation between class III β -tubulin and efficacy. In addition, there was neither a correlation between MAPT and class III β -tubulin in the xenografts nor a relationship with efficacy (the highest expression of class III β -tubulin corresponded to A6E and 286, and resistant cases had low or intermediate expression such as 194 or 253; data not shown). No mutations or polymorphisms in exons 9, 10, 11, 12, and 13 of MAPT or in exons 1, 2, 3, and 4 of class I β tubulin were found in the tested panel of cell lines (n = 11) and patient xenograft cases used in the in vivo studies (n = 9).

From Tables 2 and 3, it is interesting to note that the NO₂ and NH₂ substituents at position 2 of the benzoyl moiety and the Br substituent at position 4 on the pyrimidinyl ring apparently play an important role in the activity of these compounds. Compounds lacking either or both groups displayed weak activity. These findings were in accordance with previously reported structure-activity relationships. See, Okada, H., et al., Chem. Pharm. Bull., 39:2308-2315 (1991); and Gurulingappa, H., et al.. Bioorg. Med. Chem. Lett., 14:2213-2216 (2004). In addition, replacement of the urea moiety with thiourea (6g and 6h) had little effect on the activity. Introduction of a sulfide bridge between phenyl and pyrimidinyl rings resulted in higher activity than did an ether link. This variation was observed for compounds 6n and 7d (Tables 2 and 3), both of which showed a significant anticancer effect. For example, an increase in potency of 111-fold for Panc430, 8-fold for Panc1 and Mia- paca2, 14-fold for Panc203, 100-fold for CWR22R, and 20- fold for the LnCap cell line was obtained for compound 6n over 6m. Similarly, when compared to 7c, the activity of 7d was increased by >117-fold for Panc203, 9-fold for Miapaca2, >50-fold for Panc430, 39-fold for Panc1, and 7-fold for the LAPC-4 cell line. A large decrease in the activity of 8g and 8h (when compared to that of 6n) was also seen with the modification of sulfide to sulfoxide and sulfone. Even though 6m and 6n were effective in inhibiting tubulin assembly, their poor binding affinity for colchicine binding site indicates the existence of an alternate binding site mechanism.

The best administration schedule for compounds 6n and 7d was determined and tested in a novel platform of direct pancreas cancer xenografts taken from patient tumors. These studies showed that compounds 6n and 7d are the best candidates for further development. In addition, it was found that MAPT expression in pancreas tumor cells and tissue is a marker that thus far predicts the efficacy of compound 6n. The patient-derived xenograft model is feasible, with a high engraftment rate. Even more importantly for new drug development, the patient-derived xenografts are stable over time and passages, both genetically and from the perspective of drug sensitivity. The ability to test several drugs simultaneously allows for direct comparisons, and the availability of large amounts of tissue permits the performance of detailed biological analyses as well as the development of novel techniques for seriated pharmacodynamic assessment.

Both compounds 6n and 7d had antiproliferative activity in vitro in the low nanomolar range and showed a similar mechanism of action, with the former twice as potent as the latter as an inhibitor of tubulin assembly. This probably accounts for the greater in vivo activity of 6n. This is consistent with the finding that increased levels of MAPT were associated with resistance to these benzoylphenylurea analogues because MAP, including MAPT, strongly enhance tubulin assembly.

Another finding was that in a direct comparison in three of the cases, 6n was at least as efficacious (if not better) than docetaxel, an active anticancer agent with some activity in pancreas cancer. In comparison with other agents tested in the same patient-derived xenografts, 6n was superior to erlotinib. Additionally, the potential value of MAPT, a protein that binds to microtubule and enhances their assembly and stability and that has been linked to sensitivity to paclitaxel and docetaxel was explored. In both the cell lines and the patient-derived xenografts, there was an exquisite correlation between mRNA and protein levels, which increases the likelihood of developing a feasible diagnostic test for clinical use. Equally important, there was also a solid, inverse correlation between MAPT expression and sensitivity to all tested agents. Specifically, the same inverse correlation between MAPT and 6n/7d activity occurred with docetaxel (both in prior clinical data and in the preclinical experiments), despite the opposing mechanisms of action of benzoylphenylureas and taxanes (inhibition of tubulin assembly versus enhancement of assembly, respectively). As was noted above, the inverse correlation of MAPT expression with drug sensitivity is readily understood with the benzoylphenylurea analogues because MAP in general strongly enhance microtubule formation and thus may reduce the effect of drugs that interfere with microtubule formation.

In conclusion, the sulfur analogues of BPU of the present invention had excellent growth inhibition activity against both pancreatic and prostate cancer cell lines. Compounds 6n and 7d were both found to be more potent than compound 1. Moreover, the results obtained, specifically show that compounds 6n and 7d are the most potent in terms of cytotoxicity, growth inhibition, IC₅₀, and inhibition oftubulin polymerization, and colchicine binding in prostate cancer cell lines and pancreatic cancer cell lines.

NO₂ and NH₂ substituents at position 2 (as found in compounds 6m and compounds 7C, respectively) on the A ring (rings A-C in the BPUs, from left to right, respectively) and the Br substituent at position 4 on the C ring apparently play an important role in the activity of these compounds. BPUs lacking either or both groups exhibited weak activity. The experiments as described herein do not include an O-bridge compound possessing NO₂ and NH₂ substituents at position 2 on A ring and Br substituent at position 4 on C ring.

The data presented herein also supports structure-activity relation-("SAR") of compounds 6d, 6h, 6n, 7b, 7d, 8g, and 8h in terms of critical groups which confer activity. Specifically, NO₂ and NH₂ substituents at position 2 (as found in compounds 6n, 8g and 8h and compounds 7b and d, respectively) on the A ring and the Br substituent at position 4 on the C ring (as found in compounds 6n, 7d, 8g, and 8h) apparently play an important role in the activity of these BPUs as suggested by the results of inhibition of tubulin polymerization and colchicine binding assay.

## Claims

1. A compound represented by the structure of formula XXX: 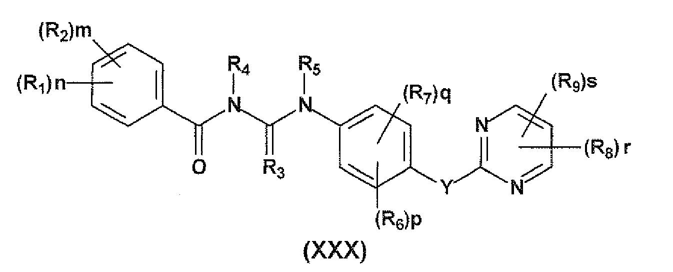 wherein R₁ and R₂ are independently H, NH₂, NO₂, Cl, Br, I, CN, NHMe, NMe₂, Me, CF₃, COOH, CONHOH, alkyl or aryl;
R₆ and R₇ are independently H, NH₂, NO₂, F, Cl, Br, I, CN, NHMe, NMe₂, CF₃, COOH, CONHOH, alkyl or aryl;
Y is S, SO or SO₂;
R₃ is O or S;
R₄ and R₅ are independently H, alkyl, aryl or -SR₁₀, wherein R₁₀ is a substituted alkyl, cycloalkyl, aryl, heterocycle, or substituted phenyl fused with a saturated or unsaturated 4- to 6-membered rings optionally containing one to three heteroatoms independently selected from N, O, and S;
R₈ and R₉ are independently H, alkyl or halogen;
n is an integer between 1-5;
m is an integer between 0-4;
r is an integer between 1-3;
s is an integer between 0-2;
q is an integer between 1-4; and
p is an integer between 0-3.

2. A compound according to claim 1 represented by the structure of formula XXXI: 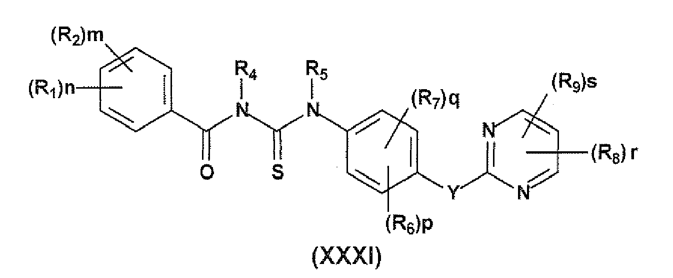 wherein R₁ and R₂ are independently H, NH₂. NO₂, F, Cl, Br, I, CN, NHMe, NMe₂, Me, CF₃, COOH, CONHOH, alkyl or aryl;
R₆ and R₇ are independently H, NH₂, NO₂, F, Br, I, CN, NHMe, NMe₂, CF₃, COOH, CONHOH, alkyl or aryl;
Y is S, SO, SO₂ or O;
R₄ and R₅ are independently H, alkyl, aryl or -SR₁₀, wherein R₁₀ is a substituted alkyl, cycloalkyl, aryl, heterocycle, or substituted phenyl fused with a saturated or unsaturated 4- to 6-membered rings optionally containing one to three heteroatoms independently selected from N, O, and S;
Rg and R₉ are independently H or alkyl or halogen;
n is an integer between 1-5;
m is an integer between 0-4;
r is an integer between 1-3;
s is an integer between 0-2;
q is an integer between 1-4 and
p is an integer between 0-3.

3. A compound according to claim 1 represented by the structure of formula XXXII: 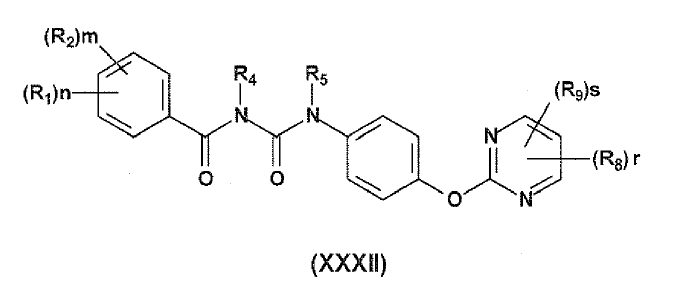 wherein, R₁ is NH₂, NHMe, NMe₂ or NO₂, and R₂ are independently H, NH₂, NO₂, CN, NHMe, NMe₂, CF₃, COOH, CONHOH, alkyl or aryl;
R₄ and R₅ are independently H, alkyl, aryl or -SR₁₀, wherein R₁₀ is a substituted alkyl, cycloalkyl, aryl, heterocycle, or substituted phenyl fused with a saturated or unsaturated 4- to 6-membered rings optionally containing one to three heteroatoms independently selected from N,
O, and S;
R₈ and R₉ are independently H, alkyl or halogen;
n is an integer between 1-5;
m is an integer between 0-4;
r is an integer between 1-3;
s is an integer between 0-2;and
wherein if R₁ is NO₂ in the ortho position, n is 1, m is 0, s is 0, r is 1, R₄ and R₅ are H, than R₈ is not iodine, Br or H in the para position.

4. The compound according to any one of claims 1 to 3, wherein R₄ and R₅ are hydrogens.

5. The compound according to claim 1 or 2, wherein R₆ is methyl.

6. The compound according to any one of claims 1 to 3, wherein R₈ is Br.

7. The compound according to any one of claims 1 to 3, wherein R₁ is NH₂ or NO₂.

8. The compound according to any one of claims 1 to 3, wherein R₈ is in the para position.

9. The compound according to any one of claims 1 to 3, wherein R₁ is in the ortho position.

10. The compound according to any one of claims 1 to 3, wherein s and m are 0.

11. A composition comprising the compound according to any one of claims 1 to 10, or its isomer, pharmaceutically acceptable salt, N-oxide, hydrate or any combination thereof; and a suitable carrier or diluent.

12. A compound according to any one of claims I to 10, or its pharmaceutical product, hydrate, pharmaceutically acceptable salt or any combination thereof, for use in the treatment of pancreatic cancer.

13. A compound according to any one of claims I to 10, or its pharmaceutical product, hydrate, pharmaceutically acceptable salt or any combination thereof, for use in the treatment of prostate cancer.

14. A compound according to any one of claims 1 to 10, or its pharmaceutical product, hydrate, pharmaceutically acceptable salt or any combination thereof, for use in inhibiting a replication of a pancreatic cancer cell.

15. A compound according to any one of claims 1 to 10, or its pharmaceutical product, hydrate, pharmaceutically acceptable salt or any combination thereof, for use in inhibiting a replication of a prostate cancer cell.

16. Use of compound according to any one of claims 1 to 10, or its pharmaceutical product, hydrate, pharmaceutically acceptable salt or any combination thereof, for the preparation of a pharmaceutical composition for the treatment of pancreatic cancer, prostate cancer or for inhibiting a replication of a pancreatic cancer cell or prostate cancer cell.
